# EUROPEAN PATENT SPECIFICATION

(11) **EP 1 187 916 B1**
(45) Date of publication and mention of the grant of the patent: **03.10.2007**
(21) Application number: 00934824.4
(22) Date of filing: 06.06.2000
(51) Int. Cl.: C12N 15/12, C07K 14/435, A61K 38/17, G01N 33/50, C12Q 1/68, C07K 16/18, C12N 5/20

(54) **IDENTIFICATION AND MOLECULAR CHARACTERISATION OF PROTEINS, EXPRESSED IN THE TICK SALIVARY GLANDS**
IDENTIFIZIERUNG UND MOLEKULARE CHARAKTERISIERUNG VON PROTEIN, DIE IN DEN SPEICHELDRÜSEN VON ZECKEN EXPRIMIERT WERDEN
IDENTIFICATION ET CARACTERISATION MOLECULAIRE DE PROTEINES EXPRIMEES DANS LES GLANDES SALIVAIRES DE LA TIQUE

(30) Priority: 09.06.1999 GB 9913425
(43) Date of publication of application: 20.03.2002
(60) Divisional application: 07109568.1
(73) Proprietor: Henogen S.A., 6041 Gosselies (BE)
(72) Inventor: GODFROID, Edmond, B-1020 Brussels (BE); BOLLEN, Alex, B-1701 Itterbeek (BE); LEBOULLE, Gérard, B-1190 Brussels (BE)
(74) Representative: Van Malderen, Joëlle
(86) International application number: PCT/BE2000/000061
(87) International publication number: WO 2000/077198

(56) References cited:
- WO-A-95/04750
- DATABASE EMBL EBI, HINXTON, UK; 14 September 1999 (1999-09-14), G. LEBOULLE: "Ixodes ricinus partial mRNA from salivary gland substractive cDNA library clone Seq24" XP002314132 retrieved from EMBL Database accession no. AJ269658 -& LEBOULLE GERARD ET AL: "Isolation of Ixodes ricinus salivary gland mRNA encoding factors induced during blood feeding." AMERICAN JOURNAL OF TROPICAL MEDICINE AND HYGIENE, vol. 66, no. 3, March 2002 (2002-03), pages 225-233, XP002314131 ISSN: 0002-9637
- G R NEEDHAM ET AL: "Characterization of Ixodid Tick salivary-gland gene products, using recombinant DNA technology" EXPERIMENTAL AND APPLIED ACAROLOGY,GB,CHAPMAN & HALL, vol. 7, 1989, pages 21-32, XP002093753 ISSN: 0168-8162
- BIOR ABDEL A D ET AL: "Differentially expressed genes in tick salivary glands." FASEB JOURNAL, vol. 13, no. 7, 23 April 1999 (1999-04-23), page A1368, XP000946819 Annual Meeting of the American Societies for Experimental Biology on Biochemistry and Molecular Biology 99;San Francisco, California, USA; May 16-20, 1999 ISSN: 0892-6638
- LUO C ET AL: "Cloning and sequence of a gene for the homologue of the stearoyl CoA desaturase from salivary glands of the tick Amblyomma americanum." INSECT MOLECULAR BIOLOGY, vol. 6, no. 3, 1997, pages 267-271, XP000952079 ISSN: 0962-1075

## Description

### FIELD OF INVENTION.

The invention relates to the molecular characterisation of DNA sequences which encode proteins expressed in the salivary glands of ticks, more particularly the *Ixodes ricinus* arthropod tick. These proteins are involved in the complex mechanism of interaction between this arthropod and its mammalian host. The invention relates to newly identified polynucleotides, polypeptides encoded by them and the use of such polynucleotides and polypeptides, and to their production.

### BACKGROUND OF THE INVENTION.

Ticks are ectoparasites which infest a large number of animals such as mammals, birds, reptiles and amphibians. They are present in almost every area of the world. The tick feeding process itself is often harmful to the host. In addition, many of these ticks are vectors of viruses, bacteria and protozoa that cause host morbidity and, in some cases, mortality, particularly in humans and livestock animals. There are three families of ticks : the *Ixodidae* or hard ticks, the *Argasidae* or soft ticks, and the *Nuttalliellidae.* The life cycle of all ticks involves four stages (egg - larva - nymph - adult). In the majority of species, as *Ixodes ricinus,* the ticks drop off the host animal after each blood meal. The larvae hatch from the eggs and climb the vegetation where they come into easy reach of passing animals. Once on the host, they attach themselves and feed on blood. Nymphs and adults feed on other hosts and employ the same methods of host seeking. Mating of adults often takes place on the host while attached and feeding. Egg laying occurs after detachment.

The tick salivary gland play an important role in the accomplishment of the blood meal and in the transmission of pathogens. During the blood meal, *Ixodidae* ticks concentrate the blood by using special mechanisms which eliminate the excess of water and ions through salivary glands. A striking modification of the morphology and the physiology of the salivary glands occurs during this blood meal. The cytoplasm and the nucleus of several salivary gland cells enhance in volume leading to an important increase in the size and the weight of the salivary glands. The messenger RNA (mRNA) synthesis is also induced, resulting in the expression of new proteins. At the end of the meal, the degeneration of the salivary glands is probably caused by the 20-hydroxyecdisone, also called "salivary degenerating factor".

The salivary gland is rich in bio-active factors : cement, enzymes, enzyme inhibitors, histamine agonist and antagonist, anticoagulant factors, modulating factors of the host immune response, prostaglandin. Some of these interactive factors are already present in the salivary glands of unfed ticks; but others, mainly proteins, are induced during the feeding phase of the blood meal. These induced-proteinic factors seem to play an important role in the modulation of the host immune response. One of these factors, a 65 kDa protein, was isolated by Brossard and co-workers (Ganapamo *et al*., 1997). This protein induces, *in vitro*, a specific CD4⁺ T cell proliferation of lymph node cells from mice infested with *I. ricinus* ticks (Ganapamo *et al*., 1997). These cells produce high levels of interleukin-4 (IL-4) and low levels of interferon-γ (IFN-γ) when they are stimulated with concanavalin-A (Con A). This suggest a T_{H}2 polarisation of the cytokine pattern (Ganapamo *et al*., 1995). The production of IL-5 and IL-10 confirms this phenomenon (Ganapamo *et al*., 1996). This polarised response could constitute favourable conditions for the transmission of some pathogens.

The inhibition of the alternative pathway of complement activation, the decrease of the synthesis of antibodies induced by thymo-dependent antigens in infested animals, and the decrease of the proliferative activity of T-lymphocytes stimulated with mitogens, contribute to the setting up of these processes (Wikel *et al*., 1996; Brossard and Wikel, 1997). Furthermore, prostaglandins (PGE₂) and salivary proteins are involved in the suppression of the immune response. In addition, it is known that some proteinic factors expressed by the salivary glands stimulate the growth of *Borrelia burgdorferi,* the causal agent of Lyme disease, which is the main human pathogen transmitted by the *Ixodidae* ticks (De Silva *et al., 1995)*

NEEDHAM G. et al. (Experimental and Applied Acarology n° 7, p. 21 a 32, 1989) describe characterization of Ixode ticks salivary - gland gene products obtained from a cDNA library.

BIOR et al. Abdel (Faseb Journal, vol. 13, n° 7, 1999) describes a project to identify and characterize tick genes expressed in salivary glands.

LUO C. et al (Insect Molecular Biology, vol. 6 (3), p. 267-271, 1997) describe the cloning and the sequencing of a gene for the homologue of a desaturaze enzyme and obtained from salivary glands of a tick.

The document WO95/04750 describes tick derived protease inhibitor polypeptide and polynucleotide sequences, a method for producing these polypeptides in a host cell and a pharmaceutical composition comprising said polypeptide.

### Summary of the invention

The present invention is related to a polynucleotide obtained from tick salivary gland and presenting more than 75%, 80%, 90%, 95%, 98 - 99% or 100% with the nucleotide sequences SEQ.ID.N° 24 or a sequence complementary thereto, as well as the corresponding polypeptide sequence.

The present invention is also related to a vector comprising the polynucleotide or the polypeptide sequences according to the invention, the cell line transfected by this vector, the antibodies raised against these polypeptides, hybridoma cell lines expressing these antibodies and a pharmaceutical composition comprising these various elements according to the invention.

A further aspect of the present invention is also related to the use of the polypeptides according to the invention for the manufacture of medicament, to produce antibodies or T-cell immune response to protect a mammal from bacteria or viruses transmitted during the blood meal of *Ixodes ricinus* and related species in the treatment and/or prevention of Lyme disease or tick encephalitis virus diseases.

The present invention is also related to a diagnostic kit for detecting a disease or susceptibility to a disease induced or transmitted by ticks, especially *Ixodes ricinus* which comprises the polynucleotide or polypeptide sequences of the invention or a phage displaying the antibodies according to the invention.

### BRIEF DESCRIPTION OF THE DRAWINGS.

### Figure 1.

RACE assay (Frohman et al., 1995) specific to Seq 16 and 24. The reverse transcription step was carried out using 10 ng of mRNAs extracted from salivary gland of engorged ticks. The brightest bands represent the cDNA fragments corresponding to the 3' end of the targeted mRNA. The amplified products were subjected to agarose gel electrophoresis followed by staining the DNA fragments by ethidium bromide. Molecular weight markers (M) were the Smart ladder (Life technologies, Rockville, Maryland, USA). Arrows indicate the position of the expected amplified products.

### Figure 2.

Differential expression analysis of the 5 full-length selected cDNAs and 9 cDNA fragment isolated in the subtractive library. PCR assays were carried out using as DNA template cDNAs obtained from a reverse transcription procedure on mRNAs extracted from salivary glands either of engorged (E) or of unfed (UF) ticks. These RNA messengers were also used as template in reverse transcription assays. Ten microliter of both PCR and RT-PCR mixture were subjected to agarose gel electrophoresis and ethidium bromide staining for the detection of amplified DNA products. [++] strongly positive; [+] positive; [-] negative.

### DESCRIPTION OF THE INVENTION.

We have characterised genes which are induced in the salivary glands of *Ixodes ricinus* during the slow-feeding phase of the blood meal. The cloning of these genes was carried out by setting up two complementary DNA (cDNA) libraries. The first one is a subtractive library based on the methodology described by Lisitsyn et al. (Science 259, 946-951,1993) and improved by Diatchenko et al. (Proc. Natl. Acad. Sci. USA 93, 6025-6030, 1996). This library cloned selectively induced mRNA during the tick feeding phase. The second library is a full-length cDNA library which has been constructed by using the basic property of mRNAs (presence of a polyA tail in its 3'end and the cap structure in its 5' end). This cDNA library permitted the cloning of full-length cDNAs, corresponding to some incomplete cDNA sequences deemed of interest, and identified in the subtractive cDNA library.

The subtractive library was set up by subtracting uninduced-cDNAs (synthesised from mRNAs expressed in the salivary glands of unfed ticks) from induced-cDNAs (synthesised from mRNAs expressed in the salivary gland at the end of the slow-feeding phase). The induced-cDNAs was digested by a restriction enzyme, divided into two aliquots, and distinctively modified by the addition of specific adapters. As for the induced-cDNAs, the uninduced cDNAs was also digested by the same restriction enzyme and then mixed in excess to each aliquot of modified induced-cDNA. Each mixture of uninduced-/induced-cDNAs was subjected to a denaturation step, immediately followed by an hybridisation step, leading to a capture of homologous induced-cDNAs by the uninduced-cDNA. Each mixture was then mixed together and subjected again to a new denaturation/hybridisation cycle. Among the hybridised cDNA molecules, this final mixture comprises induced-cDNAs with different adapters at their 5' and 3' end. These relevant cDNAs were amplified by polymerase chain reaction (PCR), using primers specific to each adapter located at each end of the cDNA molecules. The PCR products were then ligated into the pCRII™ vector by A-T cloning and cloned in an TOP-10 *E*. *coli* strain. The heterogeneity of this subtractive library was evaluated by sequencing the recombinant clones. The "induced" property of these cDNA sequences was checked by reverse transcription-PCR (RT-PCR) on mRNA extracted from salivary glands of engorged and unfed ticks. Finally, the full-length induced-cDNA was obtained by screening the expression library using, as a probe, some incomplete induced-cDNAs isolated from the subtractive library. These full-length induced DNA molecules were sequenced and compared to known polypeptide and polynucleotide sequences.

The full-length cDNA library was set up by using the strategy developed in the "CapFinder PCR cDNA Library Construction Kit" (Clontech). This library construction kit utilises the unique CapSwitch™ oligonucleotide (patent pending) in the first-strand synthesis, followed by a long-distance PCR amplification to generate high yields of full-length, double-stranded cDNAs. All commonly used cDNA synthesis methods rely on the ability of reverse transcriptase to transcribe mRNA into single stranded DNA in the first-strand reaction. However, because the reverse transcriptase cannot always transcribe the entire mRNA sequence, the 5' ends of genes tend to be under-represented in cDNA population. This is particularly true for long mRNAs, especially if the first-strand synthesis is primed with oligo(dT) primers only, or if the mRNA has a persistent secondary structure. Furthermore, the use of T4 DNA polymerase to generate blunt cDNA ends after second-strand synthesis commonly results in heterogeneous 5' ends that are 5-30 nucleotides shorter than the original mRNA (D'Alessio, 1988). In the CapFinder cDNA synthesis method, a modified oligo(dT) primer is used to prime the first-strand reaction, and the CapSwitch oligonucleotide acts as a short, extended template at the 5' end for the reverse transcriptase. When the reverse transcriptase reaches the 5' end of the mRNA, the enzyme switches templates and continues replicating to the end of the CapSwitch oligonucleotide. This switching in most cases occurs at the 7-methylguanosine cap structure, which is present at the 5' end of all eukaryotic mRNAs (Furuichi & Miura, 1975). The resulting full-length single stranded cDNA contains the complete 5' end of the mRNA as well as the sequence complementary to the CapSwitch oligonucleotide, which then serves as a universal PCR priming site (CapSwitch anchor) in the subsequent amplification. The CapSwitch-anchored single stranded cDNA is used directly (without an intervening purification step) for PCR. Only those oligo(dT)-primed single stranded cDNAs having a CapSwitch anchor sequence at the 5' end can serve as templates and be exponentially amplified using the 3' and 5' PCR primers. In most cases, incomplete cDNAs and cDNA transcribed from polyA⁻ RNA will not be recognised by the CapSwitch anchor and therefore will not be amplified.

At the end of these reactions, the full-length cDNA PCR products was ligated into the pCRII cloning vector (Invitrogen) and used for the transformation of XL2 *E*. *coli* strain. The full-length cDNA library was then screened by using, as a probe, the incomplete induced-cDNAs isolated from the subtractive library.

Eighty-nine clones of subtractive library were randomly sequenced, and their DNA and amino acid translated sequences were compared to DNA and protein databases. Among these, 27 distinct family sequences were identified, and 3 of them were selected for further characterisation of their corresponding full-length mRNA sequence. These 3 sequences matched the sequence of i) the human tissue factor pathway inhibitor (TFPI), ii) the human thrombin inhibitor gene, and iii) a snake venom zinc dependant metallopeptidase protein. These genes encode proteins which could be involved in the inhibition of the blood coagulation. The other 24 family sequences presented low or no homologies with polynucleotide and polypeptide sequences existing in databases. Screening of the full-length cDNA library using oligonucleotide probes specific to the 3 previously selected subtractive clones led to the recovery of the corresponding full-length cDNAs. Random screening of this library led to the selection of 2 other clones. One is closely homologous to an interferon-like protein, whereas the other shows homologies to the *Rattus norvegicus* leucocyte common antigen related protein.

### Definitions.

"Putative anticoagulant, anti-complement and immunomodulatory" polypeptides refer to polypeptides having the amino acid sequence encoded by the genes defined in the table. These present homologies with anticoagulant, anti-complement and immunomodulatory polypeptides already existing in databases. These polypeptides belong to the Class I and Class II sequences (see table)

"Putative anticoagulant, anti-complement and immunomodulatory" cDNAs refer to polynucleotides having the nucleotide sequence defined in the table, or allele variants thereof and/or their complements. These present homologies with anticoagulant, anti-complement and immunomodulatory polynucleotides already existing in databases. These cDNAs belong to the Class I and Class II sequences (see table)

Some polypeptide or polynucleotide sequences present low or no homologies with already existing polypeptides or polynucleotides in databases. These belong to the Class III (see table).

« Polypeptide » refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslational natural processes or may be made by synthetic methods. Modifications include acetylation, acylation, ADP-ribosylation, amidation, covalent attachment of flavin, covalent attachment of a hem moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-linkings, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino of amino acids to proteins such as arginylation, and ubiquitination. See, for instance, PROTEINS - STRUCTURE AND MOLECULAR PROPERTIES, 2nd Ed., T. E. Creighton, W. H. Freeman and Comany, New York, 1993 and Wolt, F., Posttranslational Protein Modifications : Perspectives and Prospects, pgs. 1-12 in POSTTRANSLATIONAL COVALENT MODIFICATION OF PROTEINS, B. C. Johnson, Ed., Academic Press, New York, 1983; Seifter et al., "Analysis for protein modifications and nonprotein cofactors", Meth Enzymol (1990) 182 : 626-646 and Rattan et al, "Protein Synthesis : Posttranslational Modifications and Aging", Ann NY Acad Sci (1992) 663 : 48-62.

"Polynucleotide" generally refers to any polyribonucleotide or polydeoxyribonucleotide, which may be unmodified RNA or DNA or modified RNA or DNA. "Polynucleotides" include, without limitation single-and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions. In addition, "Polynucleotide" refers to triple-stranded regions comprising RNA or DNA or both RNA and DNA. The term Polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "Polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Variant" as the term is used herein, is a Polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions identical. A variant and reference polypeptide may differ in amino acid sequence by one or more substitutions (preferably conservative), additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. Variants should retain one or more of the biological activities of the reference polypeptide. For instance, they should have similar antigenic or immunogenic activities as the reference polypeptide. Antigenicity can be tested using standard immunoblot experiments, preferably using polyclonal sera against the reference polypeptide. The immunogenicity can be tested by measuring antibody responses (using polyclonal sera generated against the variant polypeptide) against purified reference polypeptide in a standard ELISA test. Preferably, a variant would retain all of the above biological activities.

"Identity" is a measure of the identity of nucleotide sequences or amino acid sequences. In general, the sequences are aligned so that the highest order match is obtained. "Identify" *per se* has an art-recognized meaning and can be calculated using published techniques. See, e.g. : (COMPUTATIONAL MOLECULAR BIOLOGY, Lesk, A.M., ed., Oxford University Press, New York, 1988; BIOCOMPUTING : INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds, Humana Press, New Jersey, 1994; SEQUENCE ANALYSIS IN MOLECULAR BIOLOGY, von Heijne, G., Academic Press, 1987; and SEQUENCE ANALYSIS PRIMER, Gribskov, M. and Devereux, J., eds, M Stockton Press, New York, 1991). While there exist a number of methods to measure identity between two polynucleotide or polypeptide sequences, the term "identity" is well known to skilled artisans (Carillo, H., and Lipton, D., SIAM J Applied Math (1998) 48 : 1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., SIAM J Applied Math (1988) 48 : 1073. Methods to determine identity and similarity are codified in computer programs. Preferred computer program methods to determine identity and similarity between two sequences include, but are not limited to, GCG program package (Devereux, J., et al., J Molec Biol (1990) 215 : 403). Most preferably, the program used to determine identity levels was the GAP program, as was used in the Examples below.

As an illustration, by a polynucleotide having a nucleotide sequence having at least, for example, 95% "identity" to a reference nucleotide sequence is intended that the nucleotide sequence of the polynucleotide is identical to the reference sequence except that the polynucleotide sequence may include an average up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

### Polypeptides of the invention.

The present invention relates to proteins (or polypeptides) secreted by *I. ricinus* salivary glands. These polypeptides include the polypeptides encoded by the cDNAs defined in the table; as well polypeptides comprising the amino acid sequence encoded by the cDNAs defined in the table; and polypeptides comprising the amino acid sequence which have at least 75 % identity to that encoded by the cDNAs defined in the table over their entire length, and preferably at least 80 % identity, and more preferably at least 90 % identity. Those with 95-99 % are highly preferred.

The *I. ricinus* salivary gland polypeptides may be in the form of the "mature" protein or may be a part of a larger protein such as a fusion protein. It may be advantageous to include an additional amino acid sequence which contains secretory or leader sequences, pro-sequences, sequences which help in purification such as multiple histidine residues, or an additional sequence for stability during recombinant production.

Fragments of the *I. ricinus* salivary gland polypeptides are also included in the present invention. A fragment is a polypeptide having an amino acid sequence that is the same as part, but not all, of the amino acid sequence of the aforementioned *I*. *ricinus* salivary gland polypeptides. As with *I. ricinus* salivary gland polypeptides, fragment may be "free-standing" or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of the polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of the *I. ricinus* salivary gland polypeptides, except for deletion of a continuous series of residues that includes the amino terminus, or a continuous series of residues that includes the carboxyl terminus and / or transmembrane region or deletion of two continuous series of residues, one including the amino terminus and one including the carboxyl terminus. Also preferred are fragments characterised by structural or functional attributes such as fragments that comprise alpha-helix and alpha-helix forming regions, beta-sheet and beta-sheet forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate *I. ricinus* salivary gland protein activity, including those with a similar activity or an improved activity, or with a decreased undesirable activity. Also included are those that antigenic or immunogenic in an animal or in a human.

Preferably, all of these polypeptide fragments retain parts of the biological activity (for instance antigenic or immunogenic) of the *I. ricinus* salivary gland polypeptides, including antigenic activity. Variants of the defined sequence and fragments also form part of the present invention. Preferred variants are those that vary from the referents by conservative amino acid substitutions - i.e., those that substitute a residue with another of like characteristics. Typical such substitutions are among Ala, Val, Leu and Ile, among Ser and Thr; among the acidic residues Asp and Glu; among Asn and Gln, and among the basic residues Lys and Arg; or aromatic residues Phe and Tyr. Particularly preferred are variants in which several, 5-10, 1-5, or 1-2 amino acids are substituted, deleted, or added in any combination. Most preferred variants are naturally occurring allelic variants of the *I. ricinus* salivary gland polypeptide present in *I. ricinus* salivary glands.

The *I. ricinus* salivary gland polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the invention.

Another aspect of the invention relates to *I. ricinus* salivary gland cDNAs (polynucleotides). These include isolated polynucleotides which encode *I. ricinus* salivary gland polypeptides and fragments respectively, and polynucleotides closely related thereto. More specifically, *I. ricinus* salivary gland cDNAs of the invention include a polynucleotide comprising the nucleotide sequence of cDNAs defined in the table, encoding a *I. ricinus* salivary gland polypeptide. The *I. ricinus* salivary gland cDNAs further include a polynucleotide sequence that has at least 75% identity over its entire length to a nucleotide sequence encoding the *I. ricinus* salivary gland polypeptide encoded by the cDNAs defined in the table, and a polynucleotide comprising a nucleotide sequence that is at least 75% identical to that of the cDNAs defined in the table. In this regard, polynucleotides at least 80% identical are particularly preferred, and those with at least 90% are especially preferred. Furthermore, those with at least 95% are highly preferred and those with at least 98-99% are most highly preferred, with at least 99% being the most preferred, with at least 99% being the most preferred. Also included under *I*. *ricinus* salivary gland cDNAs is a nucleotide sequence which has sufficient identity to a nucleotide sequence of a cDNA defined in the table to hybridize under conditions useable for amplification or for use as a probe or marker. The invention also provides polynucleotides which are complementary to such *I. ricinus* salivary gland cDNAs.

The nucleotide sequence encoding *I. ricinus* salivary gland polypeptide encoded by the cDNAs defined in the table may be identical to the polypeptide encoding sequence contained in the genes defined in the table, or it may be a sequence, which as a result of the redundancy (degeneracy) of the genetic code, also encodes the polypeptide encoded by the genes defined in the table respectively.

When the polynucleotides of the invention are used for the recombinant production of an *I*. *ricinus* salivary gland polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro-or preproprotein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz et al, Proc Natl Acad Sci USA (1989) 86:821-824, or is an HA tag, or is glutathione-s-transferase. The polynucleotide may also contain non-coding 5' and 3' sequences, such as transcribed, non-translated sequences, splicing and polydenylation signals, ribosome binding sites and sequences that stabilize mRNA.

Further preferred embodiments are polynucleotides encoding *I. ricinus* salivary gland protein variants comprising the amino acid sequence of the *I. ricinus* salivary gland polypeptide encoded by the cDNAs defined by the table respectively in which several, 10-25, 5-10, 1-5, 1-3, 1-2 or 1 amino acid residues are substituted, deleted or added, in any combination. Most preferred variant polynucleotides are those naturally occurring *I. ricinus* sequences that encode allelic variants of the *I. ricinus* salivary gland proteins in *I. ricinus.*

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridisation will occur only if there is at least 80%, and preferably at least 90%, and more preferably at least 95%, yet even more preferably 97-99% identity between the sequences.

Polynucleotides of the invention which are identical or sufficiently identical to a nucleotide sequence of any gene defined in the table or a fragment thereof, may be used as hybridisation probes for cDNA clones encoding *I. ricinus* salivary gland polypeptides respectively and to isolate cDNA clones of other genes (including cDNAs encoding homologs and orthologs from species other than *I*. *ricinus*) that have a high sequence similarity to the *I. ricinus* salivary gland cDNAs. Such hybridisation techniques are known to those of skill in the art. Typically these nucleotide sequences are 80% identical, preferably 90% identical, more preferably 95% identical to that of the referent. The probes generally will comprise at least 15 nucleotides preferably, such probes will have at least 30 nucleotides and may have at least 50 nucleotides. Particularly preferred probes will range between 30 and 50 nucleotides. In one embodiment, to obtain a polynucleotide encoding *I. ricinus* salivary gland polypeptide, including homologs and orthologs from species other than *I. ricinus*, comprises the steps of screening an appropriate library under stringent hybridisation conditions with a labelled probe having a nucleotide sequence contained in one of the gene sequences defined by the table, or a fragment thereof; and isolating full-length cDNA clones containing said polynucleotide sequence. Thus in another aspect, *I. ricinus* salivary gland polynucleotides of the present invention further include a nucleotide sequence comprising a nucleotide sequence that hybridise under stringent condition to a nucleotide sequence having a nucleotide sequence contained in the cDNAs defined in the table, or a fragment thereof. Also included with *I. ricinus* salivary gland polypeptides are polypeptides comprising amino acid sequences encoded by nucleotide sequences obtained by the above hybridisation conditions. Such hybridisation techniques are well known to those of skill in the art. Stringent hybridisation conditions are as defined above or, alternatively, conditions under overnight incubation at 42°C in a solution comprising : 50% formamide, 5×SSC (150mM NaCl, 15mM trisodium citrate), 50mM sodium phosphate (pH7.6), 5x Denhardt's solution, 10% dextran sulfate, and 20 microgram/ml denatured, sheared salmon sperm DNA, followed by washing the filters in 0.1xSSC at about 65°C.

The polynucleotides and polypeptides of the present invention may be employed as research reagents and materials for discovery of treatments and diagnostics to animal and human disease.

### Diagnostic Assays

This invention also relates to the use of *I*. *ricinus* salivary gland polypeptides, or *I*. *ricinus* salivary gland polynucleotides, for use as diagnostic reagents.

Materials for diagnosis may be obtained from a subject's cells, such as from blood, urine, saliva, tissue biopsy.

Thus in another aspect, the present invention relates to a diagnostic kit for a disease or susceptibility to a disease which comprises:
(a) an *I*. *ricinus* salivary gland polynucleotide, preferably the nucleotide sequence of one of the gene sequences defined by the table, or a fragment thereof;
(b) a nucleotide sequence complementary to that of (a);
(c) an *I. ricinus* salivary gland polypeptide, preferably the polypeptide encoded by one of the gene sequences defined in the table, or a fragment thereof;
(d) an antibody to an *I. ricinus* salivary gland polypeptide, preferably to the polypeptide encoded by one of the gene sequences defined in the table; or
(e) a phage displaying an antibody to an *I. ricinus* salivary gland polypeptide, preferably to the polypeptide encoded by one of the cDNAs sequences defined in the table.

It will be appreciated that in any such kit, (a), (b), (c), (d) or (e) may comprise a substantial component.

The anti-*I.ricinus* salivary gland polypeptide antibodies may comprise polyclonal antibodies. Methods of preparing polyclonal antibodies are known to the skilled artisan. Polyclonal antibodies can be raised in a mammal for example, by one or more injections of an immunizing agent and, if desired, an adjuvant. Typically, the immunizing agent and/or adjuvant will be injected in the mammal by multiple subcutaneous or intraperitoneal injections. The immunizing agent may include the *I. ricinus* salivary gland polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL TDM adjuvant. The immunization protocol may be selected by one skilled in the art without undue experimentation.

The anti-*I*. *ricinus* salivary gland polypeptide antibodies may, alternatively, be monoclonal antibodies. Monoclonal antibodies may be prepared using hybridoma methods, such as those described by Kohler and Milstein, Nature. 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The immunizing agent will typically include the *I. ricinus* salivary gland polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies : Principles and Practice Academic Press, (1986) pp. 59-103).Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Rockville, Maryland. Human myeloma and mouse-human heteromyeloma cell lines also have been described for the production of human monoclonal antibodies (Kozbor, J Immunol, 133:3001 (1984) ; Brodeur et al., Monoclonal Antibody Production Techniques and Applications Marcel Dekker, Inc., New York, (1987) pp 51-63).

The culture medium in which the hybridoma cells are cultured can then be assayed for the presence of monoclonal antibodies directed against *I. ricinus* salivary gland polypeptide. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity o the monoclonal antibody can, for example, be determined by the Scatchard analysis of Munson and Pollard, Anal Biochem., 107:220 (1980).

After the desired hybridoma cells are identified, the clones may be subcloned by limiting dilution procedures and grown by standard methods (Goding, supra). Suitable culture media for this purpose include, for example, Dulbecco's Modified Eagle's Medium and RPMI-1640 medium. Alternatively the hybridoma cells may be grown in vivo as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences (U.S. Patent No. 4,816,567 ; Morrison et al., supra) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be susbtituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

In vitro methods are also suitable for preparing monovalent antibodies. Digestion of antibodies to produce fragments thereof, particularly, Fab fragments, can be accomplished using routine techniques known in the art.

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin bindings domains on their surfaces; for instance see WO92/01047. The library may be native, that is constructed from sequences obtained from an organism which has not been immunised with any of the proteins (or fragments), or may be one constructed using sequences obtained from an organism which has been exposed to the antigen of interest.

### Vaccines.

Another aspect of the invention relates to a method for inducing an immunological response in a mammal which comprises inoculating the mammal with *I. ricinus* salivary gland polypeptide or epitope-bearing fragments, analogs, outer-membrane vesicles or cells (attenuated or otherwise), adequate to produce antibody and/or T cell immune response to protect said animal from bacteria and viruses which could be transmitted during the blood meal of *I. ricinus* and related species. In particular the invention relates to the use of *I*. *ricinus* salivary gland polypeptides encoded by the cDNAs defined in the table. Yet another aspect of the invention relates to a method of inducing immunological response in a mammal which comprises, delivering *I. ricinus* salivary gland polypeptide via a vector directing expression of *I. ricinus* salivary gland polynucleotide *in vivo* in order to induce such an immunological response to produce antibody to protect said animal from diseases (Lyme disease, tick encephalitis virus disease, ....).

A further aspect of the invention relates to an immunological composition or vaccine formulation which, when introduced into a mammalian host, induces an immunological response in that mammal to a *I. ricinus* salivary gland polypeptide wherein the composition comprises a *I. ricinus* salivary gland cDNA, or *I. ricinus* salivary gland polypeptide or epitope-bearing fragments, analogs, outer-membrane vesicles or cells (attenuated or otherwise), the vaccine formulation may further comprise a suitable carrier. The *I. ricinus* salivary gland polypeptide vaccine composition is preferably administered orally or parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation iotonic with the blood of the recipient; and aqueous and non-aqueous sterile suspensions which may include suspending agents or thickening agents. The formulations may be presented in unit-dose or multi-dose containers, for example; sealed ampoules and vials and may be stored in a freeze-dried condition requiring only the addition of the sterile liquid carrier immediately prior to use. The vaccine formulation may also include adjuvant systems for enhancing the immunogenicity to the formulation, such as oil-in water systems and other systems konwn in the art. The dosage will depend on the specific activity of the vaccine and can be readily determined by routine experimentation.

Yet another aspect relates to an immunological/-vaccine formulation which comprises the polynucleotide of the invention. Such techniques are known in the art, see for example Wolff et al, Sciences, (1990) 247 : 1465-8.

### Therapeutics.

Another aspect of the invention related to the use of these *I. ricinus* salivary gland polypeptides as therapeutics agent. In considering the particular potential therapeutic areas for the likely products, the hospital disciplines cover by these products are : haematology (particularly coagulation clinics), transplantation (for immunosuppression control), rheumatology (for anti-inflammatories), and general treatment (for specific or improved anesthetics).

**Table 1 : Sequences identified in the subtractive and the cDNA full-length libraries.**

| Gene | Motifs | Similar sequences in databases | Score | Class |
|---|---|---|---|---|
| Seq. 1 | | No significative identity | | III |
| Seq. 2 | | No significative identity | | III |
| Seq. 3 | | No significative identity | | III |
| Seq. 4 | | No significative identity | | III |
| Seq. 5 | Prokariotic mbrne lipoprotein lipid attachment site | No significative identity | | III |
| Seq. 6 | | R. melioti Nitrogen fixation (fixF) | 0.00089 | III |
| | | Human Apolipoprotein B-100 | 0.0045 | III |
| | | Hu. mRNA for cAMP response element (CRE-BP1) binding prot | 0.057 | III |
| Seq. 7 | Kunitz family of serine protease inhibitor | Human BAC clone GS345D13 | 4.7¹³ | I |
| | | H. sap Tissue factor Pathway Inhibitor PI-2 | 4¹² | I |
| Seq. 8 | Prokariotic mbrne lipoprotein lipid attachment site | No significative identity | | III |
| Seq. 9 | | Pea mRNA for GTP binding prot. | 0.48 | III |
| Seq. 10 | | No significative identity | | III |
| Seq. 11 | | IL-11 R-Béta gene | 0.18 | II |
| Seq. 12 | | No significative identity | | III |
| Seq. 13 | | C. gloeosporioides cutinase gene | 0082 | III |
| Seq. 14 | | No significative identity | | III |
| Seq. 15 | | Mouse mRNA for secretory prot cont. thranspondine motifs | 0.014 | III |
| Seq. 16 | Zinc dependent metallopeptidase family | B. jararaca mRNA for jararhagin | 1.1.⁵ | I |
| | | Agkistrodon contortrix metalloproteinase precursor | 3.9⁵ | I |
| Seq. 17 | | O. aries gene for ovine INF-alpha | 0.7 | II |
| | | Interferon-omega 45 | 0.88 | II |
| | | Interferon-omega20 | 0.89 | II |
| | | RCPT PGE2 | 0.85 | III |
| | | PGE Rcpt EP2 | 0.85 | III |
| Seq. 18 | | No significative identity | | III |
| Seq. 19 | | IgG1 L chain directed against human IL2 rcpt Tac prot | 0.19 | II |
| | | Var region of light chain of MAK447/179 | 0.2 | II |
| Seq. 20 | | No significative identity | | III |
| Seq. 21 | | No significative identity | | III |
| Seq. 22 | | Mus Musculus neuroactin | 0.42 | III |
| Seq. 23 | | No significative identity | | III |
| Seq. 24 | | H. sapiens thrombin inhibitor | 2.1^{.12} | I |
| | | Cytoplasmic antiproteinase 38 kDa intracellular serine prot. | 2.3^{.12} | I |
| Seq. 25 | | No significative identity | | III |
| Seq. 26 | | No significative identity | | III |
| Seq.27 | | Mus musculus transcription factor ELF3 (fasta) | 0.053 | III |
| Seq. 28 | | Homo sapiens putative interferon-related protein (SM15) mRNA | 1.70E-22 | II |
| Seq. 29 | | R.norvegicus mRNA for leucocyle common antigen-related protein | 4.80E.09 | II |

| | | | | |
|---|---|---|---|---|
| Class I : putative anticoagulant homologs ; Class II : putative immunomodulatory homologs ; Class III : low or no homologies found in the databases. | | | | |

**TABLE 2. Biological characteristics of the selected clones**

| The full sequences of S selected clones were compared with EMBL/GenBank data bases using the tFasta and Blastp algorithms. These sequences were analysed for the presence of a Kozak consensus sequence indicated in the column entitled "Nucleotide in position -3". By applying the algorithm of the GCG*program the deduced amino sequences were analysed for the presence of specific protein units or motifs (algorithm motif) and for the presence of a signal peptide sequence (von Heijne and McGeoch analysis). | | | | | | | |
|---|---|---|---|---|---|---|---|
| Clone | Full-length sequences similarity to databases | (Fasla/Blastp Scores^{a} | ORF (aa) | Motifs | Signal peptide scores^{b} | Sp length/ Prob. | Nucleotide in position -3^{c} |
| Seq28 | *Homo sapiens* putative interferon- related gene (SKMc15) [U09585] | 1.8.10⁻³⁶/1.10⁻⁷¹ | 426 | | d 5.4/F^{e} | 48 aa /8.4.10⁻¹ | G |
| Seq29 | *R. norvegicus* leukocyte common antigen (LAR) mRNA [X83546] | 7,8.10⁻¹¹/N | 274 | | 10,2/S | 18 aa/7.4.10⁻⁷ | A |
| Seq16 | Mouse mRNA for secretory protein containing thrombospondin motives [D67076] | 0,002/6.10⁻⁷ | 489 | Metallopeptidase | 7.9/S | 19 aa/7,4.10⁻⁴ | G |
| Seq24 | Pig leukocyte elastase inhibitor mRNA. [P80229] | 0/7.10⁻⁶⁷ | 378 | Serpin | 8,5/S | 51 aa/3,28.10⁻³ A | |
| Seq7 | Human Tissue Factor Pathway Inhibitor [P48307] | 4,8.10⁻¹²/2.10⁻³ | 87 | Kunitz | 6.5/S | 19 aa/4.8.10⁻⁴ | G |

| | | | | | | | |
|---|---|---|---|---|---|---|---|
| ^{a} No score (N) ^{b} Succeeded (S) and Failed (F) ^{c} Guanine (G) and Adenine (A) ^{d} von Heijne analysis ^{e} McGeoch analysis * GCG = Genetic Computer Group, Madison Wisconsin, 10.0 version in Unix, January 1999. | | | | | | | |

### EXAMPLES

### Biological materials used in this study.

The salivary glands of 5 day engorged or unfed free of pathogen Ixodes ricinus female adult ticks were used in this work. When removed, these glands were immediately frozen in liquid nitrogen and stored at -80°C. To extract RNA messengers (mRNA), the salivary glands were crushed in liquid nitrogen using a mortar and a pestle. The mRNAs were purified by using an oligo-dT cellulose (Fast Track 2.0 kit, Invitrogen, Groningen, The Netherlands). Two micrograms of mRNAs were extracted from 200 salivary glands of fed ticks; and 1.5 µg of mRNAs were also extracted from 1,000 salivary glands of unfed ticks.

### Example 1 : Construction of a Representational Difference Analysis (RDA) subtractive library.

All procedures were performed as described by Hubank and Schatz (1994). Double-stranded cDNAs were synthesised using the Superscript Choice System (Life Technologies, Rockville, Maryland, USA). The cDNAs were digested with DpnII restriction enzyme, ligated to R-linkers, amplified with R-24 primers (Hubank and Schatz, 1994), and finally digested again with the same enzyme to generate a "tester" pool consisting of cDNAs from salivary glands of fed ticks and a "driver" pool consisting of cDNAs from salivary glands of unfed ticks. The first round of the subtractive hybridisation process used a tester/driver ratio of 1:100.The second and third rounds utilised a ratio of 1:400 and 1:200,000, respectively. After three cycles of subtraction and amplification, the DpnII-digested differential products were subdivided according to size into 4 different fractions on a 1.7% electrophoresis agarose gel, and subcloned into the BamHI site of the pTZ19r cloning vector. The ligated product was used to transform TOP-10 E. coli competent cells (Invitrogen, Groningen, The Netherlands). Nine thousand six hundred clones of this subtractive library were randomly selected, and individually put in 100 microplates and stored at -80°C. This subtractive library was analysed by sequencing 89 randomly chosen clones, using M13 forward and reverse primers specific to a region located in the pTl9r cloning vector. The DNA sequences of these 89 clones were compared, and 27 distinct family sequences were identified. Homology of these sequences to sequences existing in databases is presented in Table 1. The subtractive sequences 1 to 27 are presented in the sequence-listing file (except for sequences 16 and 24 whose complete mRNA sequences are presented; see also Example 2). Three sequences (Seq 7, 16 and 24) were selected for further characterisation of their corresponding full-length mRNA sequence. These 3 sequences matched the sequence of i) the human tissue factor pathway inhibitor (TFPI), ii) a snake venom zinc dependant metallopeptidase protein, and iii) the human thrombin inhibitor protein, corresponding to Seq 7, 16 and 24, respectively. These genes encode proteins which could be involved in the inhibition of the blood coagulation.

### Example 2 : Construction of the full length cDNA library and recovery of full length cDNAs sequences by screening of this full length cDNA library.

This library was set up using mRNAs extracted from salivary glands of engorged ticks. The mRNAs (80 ng) were subjected to reverse transcription using a degenerated oligo-dT primer (5'-A(T)30VN-3'), the SmartTM oligonucleotide (Clontech, Palo Alto, USA), and tthe Superscript II reverse transcriptase (Life Technologies, Rockville, Maryland, USA). The single strand cDNA mixture was used as template in a hot start PCR assay including the LA Taq polymerase (Takara, Shiga, Japan), the modified oligo-dT primer and a 3'-'Smart' primer specific to a region located at the 5' end of the SmartTM oligonucleotide. The PCR protocol applied was : 1 min at 95°C, followed by 25 sec at 95°C / 5 min at 68°C, 25 times, and 10 min at 72°C. The amplified double stranded cDNA mixture was purified with a Centricon 30 concentrator (Millipore, Bedford, USA). The cDNAs were divided into 4 fractions ranging from 0.3 to 0.6 kb, 0.6 to 1 kb, 1 kb to 2 kb and 2 kb to 4 kb on a 0,8% high grade agarose electrophoresis gel and recovered separately by using the Qiaex II extraction kit (Qiagen, Hilden, Germany). The 4 fractions were ligated individually into the pCRII cloning vector included in the TOPO cloning kit (Invitrogen, Groningen, The Netherlands). The ligated fractions were then used to transform XL2-Blue ultracompetent E. coli cells (Stratagene, Heidelburg, Germany). The resulted recombinant clones were stored individually in microplates at -80°C. Ten clones were randomly chosen for partial or complete sequencing. As a result of this procedure, 2 cDNA sequences (Seq 28 and Seq 29, see Table 1) were selected for their homology to sequence databases. One is closely homologous to an interferon-like protein (Seq 28), whereas the other shows homologies to the *Rattus norvegicus* leucocyte common antigen-related protein (Seq 29).

The 4 different fractions of the full-length cDNA library were screened with radio-labelled oligonucleotide probes specific to selected clones identified in the substractive cDNA librairy. The labelling of these oligo probes was performed as described in Current Protocols in Molecular Biology (Ausubel et al, 1995, J. Wiley and sons, Eds). These 4 different fractions were then plated on nitrocellulose membranes and grown overnight at 37°C. These membranes were denatured in NaOH 0.2M / NaCl 1.5M, neutralised in Tris 0.5M pH 7.5-NaCl 1.5M and fixed in 2X SSC (NaCl 0.3 M/ Citric Acid Trisodium di-hydrated 0.03 M). The membranes were heated for 90 min. at 80°C, incubated in a pre-hybridisation solution (SSC 6X, Denhardt's 10X, SDS 0,1%) at 55°C for 90 min., and finally put overnight in a preheated hybridisation solution containing a specific radio-labelled oligonucleotide probe at 55 °C. The hybridised membranes were washed 3 times in a SSC 6X solution at 55 °C for 10 min, dried and exposed on Kodak X-OMAT film overnight at -80°C. The full lenght cDNA library was also analysed by sequencing a set of clones. The resulted DNA sequences were compared to ENBL/Gene Bank databases and were used to set up oligonucleotide probes to recover other corresponding clones. In this way, the complete consensus mRNA sequence of the Seq 28 and 29 was confirmed by the recovery of two other clones corresponding to these sequences. Only one full-length cDNA clone corresponding to the subtractive clone 16 was isolated. Therefore, to identify the complete sequence of the Seq 16 and 24, the Rapid Amplification of cDNA Ends (RACE) method was applied.

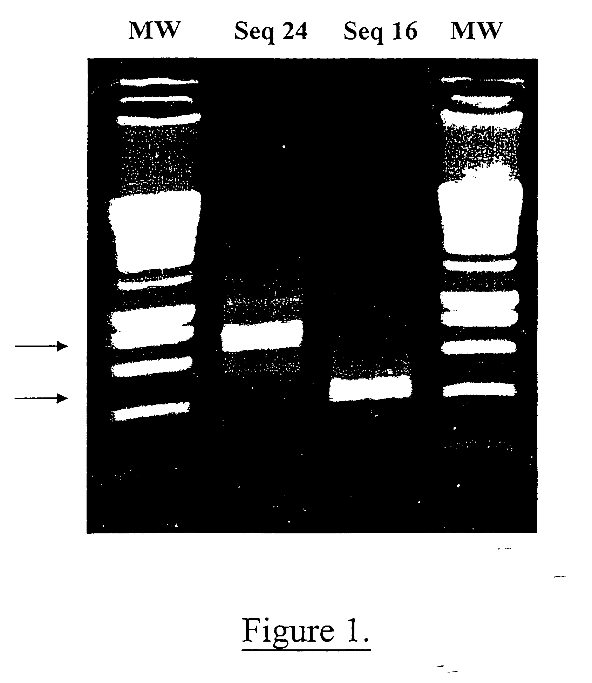

The RACE methodology was performed as described by Frohman et al. (1995). The reverse transcription step was carried out using 10 ng of mRNAs extracted from salivary gland of engorged ticks and the Thermoscript Reverse transcriptase (Life technologies, Rockville, Maryland, USA). All gene specific primers (GSP) had an 18 base length with a 61% G/C ratio. The amplified products were subjected to an agarose gel electrophoresis and recovered by using an isotachophorese procedure. The cDNAs were cloned into the pCRII-TOPO cloning vector (Invitrogen, Groningen, The Netherlands). To identify the consensus cDNA sequence, different clones were sequenced., and their sequence was compared to their known corresponding sequence. Therefore, the complete cDNA sequences of the clones 16 and 24 isolated in the subtractive library were obtained by this RACE procedure (figure 1).

### Example 3 : Analysis of the full sequences of 5 selected clones.

The sequences of selected clones (Seq. 7, 16, 24, 28 and 29) allowed identification of the open reading frames, from which the amino sequence were deduced. These potential translation products have a size between 87 and 489 amino acids (see table 2). In order to evaluate, in silico, their respective properties, the amino acid sequences and the nucleotide sequences of said 5 open frames were compared with the databases using the tFasta and Blastp algorithms. These comparisons show that Seq. 7 is highly homologous to the human Tissue Factor Pathway Inhibitor (TFPI). TFPI is an inhibitor of serine proteases having 3 domains of the Kunitz Protease Inhibitor (KPI) type. Each of these units or motifs has a particular affinity for different types of proteases. The first and second KPI domains are responsible for the respective inhibition of VIIa and Xa coagulation factors. The third KPI domain apparently has no inhibitory activity. It should be noted that the sequence coded by the Seq. 7 clone is homologous to the regio of the first KPI domain of TFPI and that the KPI is perfectly kept therein. This similarity suggests that the Seq. 7 protein is a potential factor VIIa inhibitor.

The amino sequence deduced from the Seq. 28 clone has a great homology with 3 database sequences, namely : mouse TIS7 protein, rat PC4 protein and human SKMc 15 protein. These 3 proteins are described as putative interferon type factors. They possess very well conserved regions of the B2 interferon protein. Therefore, it is proposed that the Seq. 28 protein has immunomodulatory activity.

Sequences 16 and 24 were compared with databases thus showing their homology respectively with *Gloydius halys* (sub-order of ophidians) M12b metallopeptidase and porcine elastase inhibitor belonging to the super-family of the serine protease inhibitors (Serpin). The amino sequences of these 2 clones also have specific units of said families. It is proposed that said proteins have anticoagulant and immuno-modulatory properties.

Finally, the Seq. 29 clone has a weak homology with the *R. norvegicus* leucocyte common antigen (LAR). The latter is an adhesion molecule. It is thus possible that the Seq. 29 protein has immunomodulatory properties related to those expressed by the LAR protein.

Due to their potential properties, most of the proteins examined are expected to be secreted in the tick saliva during the blood meal. Accordingly, tests were made for finding the presence of a signal peptide at the beginning of the deduced amino sequences. All of the results by the Von Heijne analysis method were positive. By the McGeoch method, signal peptide sequences were detected for the Seq. 7, 16, 24 and 29 deduced amino sequences. It seems that said proteins are secreted in the tick salivary gland. Furthermore, the presence of a Kozak consensus sequence was observed upstream of the coding sequences of all examined clones. This indicates that their mRNAs potentially could be translated to proteins.

### Example 4 : Evaluation of the differential expression of the cDNA clones isolated in the subtractive and full-length cDNA libraries.

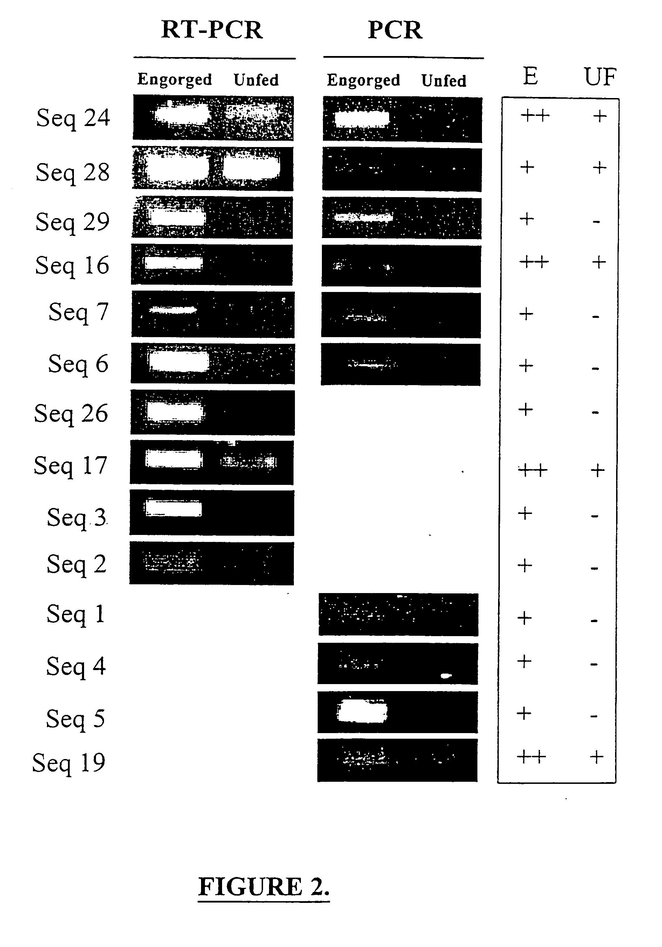

The differential expression of the mRNAs corresponding to the 5 full-length selected clones (Seq 7, 16, 24, 28 and 29) and of 9 subtractive clones was assessed using a PCR and a RT-PCR assays (figure 2).

The PCR assays were carried out using as DNA template cDNAs obtained from a reverse transcription procedure on mRNAs extracted from salivary glands either of engorged or of unfed ticks. Each PCR assay included pair of primers specific to each target subtractive or cDNAs full-length sequence. PCR assays were performed in a final volume of 50 µl containing 1µM primers, 0.2 mM deoxynucleotide (dATP, dCTP, dGTP and dTTP; Boehringer Mannheim GmbH, Mannheim, Germany), PCR buffer (10 mM Tris-HCl, 50 mM KCl , 2.5 mM. MgCl2 , pH 8.3) and 2.5 U of Taq DNA polymerase (Boehringer mannheim GmbH, Mannheim, Germany). DNA samples were amplified for 35 cycles under the following conditions : 94°C for 1 min., 72 °C for 1 min. and 64 °C for 1 min, followed by a final elongation step of 72 °C for 7 min.

The RT-PCR assay was carried out on the 5 selected full-length cDNA clones and on 5 cDNA subtractive clones. The mRNAs used as template in the reverse transcription assay was extracted from salivary glands of engorged and unfed I. ricinus ticks. The reverse transcription assays were performed using a specific primer (that target one the selected sequences) and the "Thermoscript Reverse transcriptase" (Life technologies, Rockville, Maryland, USA) at 60°C for 50 min. Each PCR assay utilised the reverse transcription specific primer and an another specific primer. The PCR assays were performed in a final volume of 50 µl containing 1 µM primers, 0.2 mM deoxynucleotide (dATP, dCTP, dGTP and dTTP; Boehringer Mannheim GmbH, Mannheim, Germany), PCR buffer (10 mM Tris-HCl, 50 mM KCl , 2.5 mM. MgCl2 , pH 8.3) and 2.5 U of Expand High Fidelity polymerase (Roche, Bruxelles, Belgium). Single stranded DNA samples were amplified for 30 cycles under the following conditions : 95°C for 1 min., 72 °C for 30 sec. and 60 °C for 1 min, followed by a final elongation step of 72 °C for 7 min. The figure 2 shows that the expression of the selected sequences is induced in salivary glands of 5 day engorged ticks, except for the sequence 28 that is expressed at a similar level in salivary glands of engorged and unfed ticks. The expression of the other mRNAs could be either induced specifically or increased during the blood meal.

### Example 5 : Expression of recombinant proteins in mammal cells.

The study of the properties of isolated sequences involves the expression thereof in expression systems allowing large amounts of proteins encoded by these sequences to be produced and purified. These experiments are described hereafter as well as in example 6.

5.1. Sub-cloning of the sequences in the p CDNA3.1-His/V5 (Invitrogen) vector.

The DNA sequences of the 5 selected clones (Seq. 7, 16, 24, 28 and 29) were transferred into the p CDNA3.1 His/V5 expression vector. Said vector allows the expression of heterologous proteins fused to a tail of 6 histidines as well as to the V5 epitope in eucaryotic cells. The different DNAs were produced by RT-PCR by using primers specific to the corresponding clones. These primers were constructed so as to remove the stop codon of each open reading frame or phase in order to allow the protein to be fused to the 6xHIS/Epitope V5 tail. In addition, the primers contained restriction sites adapted to the cloning in the expression vector. Care was taken to use, when amplifying, a high fidelity (Promega) Pfu polymerase.

The transient expression of the Seq. 16 and 24 recombinant proteins was measured after transfection of the Seq. 16 and Seq 24 - pCDNA3.1-His/V5 constructions in COSI cells, using Fugen 6 (Boehringer). The protein extracts of the culture media corresponding to times 24, 48 and 72 hours after transfection were analysed on acrylamide gel by staining with Coomassie blue or by Western blot using on the one hand an anti-6x histidine antibody or on the other hand Nickel chelate beads coupled to alcaline phosphatase. These analyses showed the expression of said proteins in the cell culture media.

### Example 6 : Expression of proteins in E. coli

### 6.1. Insertion of coding sequences into the pMAL-C2E expression vector.

It also was decided to express various proteins in bacteria by using the pMAL-C2E (NEB) vector. Said vector expresses the various proteins fused with the Maltose-Binding-Protein (MBP). The protein of interest then could be separated from the MBP thanks to a site separating the MBP from the protein, said site being specific to protease enterokinase.

In order to express optimally the 5 sequences examined, using the p MAL-C2E vector, PCR primer pairs complementary to 20 bases located upstream of the STOP codon and to 20 bases located downstream of the ATG of the open reading frame or phase were constructed. In that way, the amplified CDNA fragments only comprised the coding sequence of the target mRNA provided with its stop codon. The protein of interest was fused to MBP by its N-terminal end. On the other hand, since these primers contained specific restriction sites specific to the expression vector, it was possible to effect direct cloning of the CDNAs. The use of Pfu DNA polymerase (Promepa) made it possible to amplify the cDNAs without having to fear for errors introduced into the amplified sequences.

The coding sequences of clones Seq 7, 16, 24 and 29 were reconstructed in that way Competent TG1 cells of E. *coli* were transferred using these constructions. Enzymatic digestions of these mini-preparations of plasmidic DNA made it possible to check that the majority of clones Seq. 7, 16, 24 and 29-p-MALC2-E effectively were recombinant.

### 6.2. Expression of recombinant proteins.

Starting from various constructions cloned in *E. coli* TGI, the study of the expression of recombinant proteins fused with MBP was initiated for all sequences of interest (i.e. Seq. 7, 16, 24 and 29) except for Seq. 28. The culture of representative clones of Seq. 7, 16, 24 and 29 as well as negative controls (non recombinant plasmids) were undertaken to induce the expression of recombinant proteins therein. These cultures were centrifuged and the sediments (culots) were separated from the media for being suspended in 15mM pH7.5 Tris and passed to the French press. The analysis of these samples by (10%) acrylamide gel coloured with Coomassie blue or by Western Blot using rabbit anti-MBP antibodies, showed the expression of recombinant proteins Seq. 7 (~50kDa), Seq. 16 (~92kDA), Seq 24 (~80kDA) and Seq 29 (~67kDa).

### Example 7 : Production of antibodies.

The Seq. 7, 16 and 24 protein were injected into groups of 4 mice with the purpose of producing antibodies directed against said proteins. The first antigen injected was made with the complete Freund adjuvant. Two weeks later, a recall injection was made with incomplete Freund adjuvant. The sera of mice injected with Seq. 16 provided positive tests for anti-MBP antibodies.

### SEQUENCE LISTING

(1) INFORMATION FOR SEQ ID N°: 1
   (i) SEQUENCE CHARACTERISTICS :
      (a) LENGTH : 194 base pairs
      (b) TYPE : nucleic acid
      (c) STRANDEDNESS : single
      (d) TOPOLOGY : linear
   (ii) MOLECULE TYPE : cDNA
   (iii)SEQUENCE DESCRIPTION : SEQ ID N°1:
   1 ATACCTTCCA CTTGTAGCCC TTCCTCATCC GATATGGTGA CGGATGCCAT
   51 TGCATCCTCG TCGTGGAAGA GGTCCTCTTC TAAATAAGAC CCATCCATAT
   101 ATGTGTGTTT GCGAATGCCG TCGACGTAGC TCCTGACTAG AAACTCGTCG
   151 GCTAGGACAG AACTTTTCTT CAGGTTTAGC GTAATGTCCT CGTT
(2) INFORMATION FOR SEQ ID N°: 2
   (i) SEQUENCE CHARACTERISTICS :
      (a) LENGTH : 607 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N° 2:
   1 TACCNGGGAA TCCAAAACCA ATTTTTATTG GAACTTCCAC GTCTTCTTCA
   51 AGGCGGTGGC ACCTCTGCAT TTATGAAGTT CGTCTTGGCA TTTTATTTTT
   101 TGCTTCTTTC ATTGCRGAAC TCGCAAATGC ACTTCCCGTG CTTGTCGCAT
   151 TTCGCCCCAA AAGCGCATGG CATTCCTTCC GGCAGATTAA CTTTTTCAAA
   201 TTCACGGTTC TGAACCAATA ATAGATCGTG GCAATGTTTG TGCTGTTTGC
   251  GATTTGCAAA CCAGCTGTAG CCACCATTGG ACTCAAAGGT GCGCACAACA
   301  TGGCGCCGAA CTGTGAAAAA CAAATTAAGG CTNCTTTGTA ATAACGCTAG
   351  TCTTGGTACG CCGTTAGAGG TCGATGTCGC GCCTCGCGAT TGCAAAGTCA
   401  CTTGCACTTA TCAAGCTCCT GGAGAAAAAT GGGTGCAACG GGGGGATCAG
   451  CGTTTGTACT TGCAAACATT TGTGGAGACG GTAAACCWGT ATTTCGCGGA
   501  ACTCAGATGC TCCAGCGTGA AGCTCGTCTT AATAAAAGTT GTAAATTCGA
   551  GTATNGATGA AGAACTGAAA TTCGAGGCAT TTAGAAACAC CACGAGAAGC
   601  AGCGGAA
(3) INFORMATION FOR SEQ ID N°: 3
   (i) SEQUENCE CHARACTERISTICS :
      (a) LENGTH : 259 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°3 :
   1  GATCCTACGC CTGAAAATGA GTGTCCATCG TCTTCACATA GTGCCACATT
   51  GTAATTGGTA CAAGCTCCAT TTTCGTCAGC GCTGTTTGTT ATGCTGCCGC
   101  CTACTTTTCC TTCGGCACTC CATAAGTTAA ACCCTGTCAT TATAAGTGTG
   151  ATTGCCGTAT CTCGGCTGAA TGGGTTCCAT TTTTCTCTTA AATAATCACG
   201  TGTCCATATT CCATGTATTG TGTTCATGAG TATGTGATTC TCATCGTATA
   251  TCTTCGCCT
(4) INFORMATION FOR SEQ ID N°: 4
   (i) SEQUENCE CHARACTERISTICS :
      (a) LENGTH : 170 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°4 :
   1  CCACTCGAAA ATGGAGGCTT TGAAACATTT CAGTACCCCT GTGAACTCTG
   51  GCTTTGCAAT GTAACAGCAA AAACACTTAC AGTTGAAGGG TGCAGTGTCA
   101  GACGCTATGG AAGTTGCATC CACGAGCACR ACCCTGATTA CTACTGGCCA
   151  CGTTGCTRTC CGGGTCGTCC
(5) INFORMATION FOR SEQ ID N°: 5
   (i) SEQUENCE CHARACTERISTICS :
      (a) LENGTH : 168 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°5 :
   1  GTATGTTACC ATGTCCAACC CGGTTATTAA ATACACCAAG TCGTAGGATT
   51  TGTAGGCAGC TGCATTGCCC TTGACGTACT CTCTCAACGT TGCCAAGGAC
   101  TCAGGCCCAT AAATGTAGTG GGGTTGACCT TGAACTCTTC GTAAAAAGCG
   151  TTCTTTCTCC GTCGTGAG
(6) INFORMATION FOR SEQ ID N°: 6
   (i) SEQUENCE CHARACTERISTICS :
      (a) LENGTH : 247 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°6 :
   1  CCGAAMATAA AACTTAGTCT CACCAATATA CGTTTGCCTA ACGCGAAGGA
   51  ACAGGCACAA ATATACTACG AGCACGACAT TCTCAAGAAC ACGGTTCACG
   101  GAGTGTGGAC GAGAATTCAC TCAAAATATC CGTTCCCTGA AGATGAGGGA
   151  ATTACACTGA TAATGACAGG GTTTGATTTA TGGAGTGCCG ATTTAACTGT
   201  AGGCGGCACC ATAACAAACA GCGCTGAGAA AAGCGGAGCT TGTACGA
(7) INFORMATION FOR SEQ ID N°: 7
   (i) SEQUENCE CHARACTERISTICS :
      (a) LENGTH: 261 base pairs
      (b) TYPE : nucleic acid
      (c) STRANDEDNESS : single
      (d) TOPOLOGY : linear
   (ii) MOLECULE TYPE : cDNA
   (iii) FEATURE:
      (a) NAME/KEY : CDS
      (b) LOCATION: 1 .. 261
   (iv) SEQUENCE DESCRIPTION : SEQ ID N°7
   1 ATG CCT TTT ATT TTC GTG GTG AGC TTA GTC ATT GTG GCC TGC ATC GTG GTA GAC ACA
   Met Pro Phe Ile Phe Val Val Ser Leu Val Ile Val Ala Cys Ile Val Val Asp Thr
   58 GCC AAC CAC AAA GGT AGA GGG CGG CCT GCG AAG TGT AAA CTT CCT CCG GAC GAC GGA
   Ala Asn His Lys Gly Arg Gly Arg Pro Ala Lys Cys Lys Leu Pro Pro Asp Asp Gly
   115 CCA TGC AGA GCA CGA ATT CCG AGT TAC TAC TTT GAT AGA AAA ACC AAA ACG TGC AAG
   Pro Cys Arg Ala Arg Ile Pro Ser Tyr Tyr Phe Asp Arg Lys Thr Lys Thr Cys Lys
   172 GAG TTT ATG TAT GGC GGA TGC GAA GGA AAC GAA AAC AAT TTT GAA AAC ATA ACT ACG
   Glu Phe Met Tyr Gly Gly Cys Glu Gly Asn Glu Asn Asn Phe Glu Asn Ile Thr Thr
   229 TGC CAA GAG GAA TGC AGA GCA AAA AAA GTC TAG
   Cys Gln Glu Glu Cys Arg Ala Lys Lys Val End
(8) INFORMATION FOR SEQ ID N°: 8
   (i) SEQUENCE CHARACTERISTICS :
      (a) LENGTH : 292 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°8 :
   1   CATCGMAGCC ATAGTATATT TTGCACTTGT CTTCCGTTTC GTCGTAGTAG
   51  GACCGATTCC ACATTGTAGT ACACCAGTCA CTTATATCCT GCGGGCGGTG
   101 CTTGCATTTG TCCTGAACAA ATCTTCCACA GCGCTTGTCG CACGCCTCCT
   151 GGGAATAGAA CGCGTTCTCT CCTCCGCATC TCCATTTGGA ATCATAGAAA
   201 CATCTTTCAG TTTGAATATT GTAGCGATAA TAATCGGTAT CAGTTTCTTT
   251 GCATGGTCCT GGGAGGGGTT TGGCGCAGGG GCCGTATTCA GG
(9) INFORMATION FOR SEQ ID N°: 9
   (i) SEQUENCE CHARACTERISTICS :
      (a) LENGTH : 270 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°9 :
   1   GGTAATAGTT GTCAAATTCC ATTAATGTAT CCTGAAATGT GACCATATCT
   51  TTGTTTCCCC TGTAAAATCT CATAAAAGGC TGTGTGTTTT CCTTAAGAAG
   101 TGTAACAGCC ACGATGGTCA ATCTCACGGA TGGATGTGTG ACACTTTTAT
   151 ATCTCAGGTT TGCCGACATT GCCATTACAG ATAAATAGTT GATAATTTCT
   201 TTCTTGTTAT AGTTGTAAGC AGCGCATGTT GTTGCATCAA GCACCACATG
   251 CACTTCAGGC AATATGGTTT
(10) INFORMATION FOR SEQ ID N°: 10
   (i) SEQUENCE CHARACTERISTICS :
      (a) LENGTH : 3 16 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°10 :
(11) INFORMATION FOR SEQ ID N°: 11
   (i) SEQUENCE CHARACTERISTICS :
      (a) LENGTH : 241 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°11 :
   1 AAGGTAGCAA GGGTGGTAGG CTTTCCTCAC AAAGAGTCTG GCTTCCGTGA
   51 TAACCATATC CATTCCTCAC CGTATACCCG TCATCCAACG TCAATTGTGT
   101 TACAAGGCAG ATAATGTCAA AATGGCTCTG GTCCCTATAA TAGTCGGATA
   151 ATGTAGAAAT CGCTCCATGT GGCCAAATAG ATGTTCCTCT TTCATACTGT
   201 TTTAACTTTA ATTGTAGGTC CGCCTCGTTC TCGAGGTATG T
(12) INFORMATION FOR SEQ ID N°: 12
   (i) SEQUENCE CHARACTERISTICS :
      (a) LENGTH : 636 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°12 :
   1   TTCCCCNAAT TGGCCTTGCG ANNCTTGCAA GTCGACNCTA GAGGCTCCGA
   51  AGATGGACAG ATTGCGCATG AAATATTTGA AATCGAGCAG AATGGTGATT
   101 TTAGGAGCGA TTATATTGTG CCACCCAGTT TGAAAGTGCA AGAACGCACA
   151 GTGGTTTACC GTAACAAGTA CACCAGAGTT CCTGTAAATT TTACCGTCGA
   201 AGTTGCCATG CTGATTGATA AGTATTTATA CWAGGAGTTC AAGAACGAGA
   251 GCCACATCGT ACCGTACCTG GCTATGATAC TGACTTTGAT AAATCTGAGG
   301 TATGCCGACA CACATGACCC GTACATCCAG TTTCTTCTCA CACAAGTGTT
   351 CGTGGGGAAW WCTGGCGATC ATATGGGCCA CATGCCCTTC CGACGAGCGT
   401 TCTTGTTCAG GCGCCGGCAT TATGCGCAGT TTAGGCCCAA TMACACCTTC
   451 CACTTGTAAT TCTCCGTTGT TGGATAGTGT AAGTGAGGCC ATTGCATCAG
   501 CATCGTGGAA GARGCCTTCC TCCAAGTAGG AACCGCCCAT TTAGGTTTGC
   551 TTTCCCAATC CGCCAATTTA ANTTTTAAAA AAAATTCCCC CCCCAAAAAT
   601 TAATTTTTTT TAAAGGTGGA TTGTGATTTC TCCGTT
(13) INFORMATION FOR SEQ ID N°: 13
   (i) SEQUENCE CHARACTERISTICS :
      (a) LENGTH : 432 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°13 :
   1   GATCCCAAAA GTGCCCCTGG ARCGACGGTT ACATCATGAG CTACGTCATA
   51  AACTTCAAAA ACCACTTCAA ATTTTCTCCC TGCTGTGTAG AATCAATTCG
   101 ATTCGTCGCA CGAGAGCGGG ACTGCCTCTA CAAAGTCAAT GCCAAGGATG
   151 CTGTAAAAAG CCTAATATCT CTGCCCGGAT TTAGGATATC GCCAACGAGT
   201 TTCTGTCAAT TTATGCATCC GCTTTACCGC GGTGTCCATA GCGATAAGAA
   251 AGCAGGTCTG TCCGATTGCG TACAGACGTG TAGAACGGCC AAAAATCGAC
   301 GAGGAGGCTA CCATTCATGG ATTCACGCGG CACTTGACGG GGTTCCTTGC
   351 GACAAGAGAA ACCCCAAGAA GGCCTGCATA AACGGGAAAT GCACCCTCCT
   401 TAAGAGCATG CCCCACAGAA CGTACCGGGA AT
(14) INFORMATION FOR SEQ ID N°: 14
   (i) SEQUENCE CHARACTERISTICS :
      (a) LENGTH : 466 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION: SEQ ID N° 14:
   1 AGGGCGTTCT TTGCTTYACA GGGAACRGCA TATGGGCCAC GTGACCTTCC
   51 AATGACCGCT CCAAATCTGG CATAGGTTGA AYTCGCAAGT CGTGGCGCAG
   101 CAGGCCTYCC ACATTCACTC CATCCTCGTC TTTTAGGATG ACTGCCGCCA
   151 TTTGTTTTGT ATCGTGGTAC AGGTGTTTGT TATGGTCCGA GCCGTCGACA
   201 TAAGTATTGA CCAACGATCG GCCGAATGAT TACGGCTCAC CAAACACATC
   251 AAATACCCCC GTCAAGTCAA GAGCTGGAAG CACAAAGCAT AGTATGTACA
   301 AGATACCCTT GGAAATCTTT CCCGAAGTTC ACCTTGTGGT GGACAGCACA
   351 TTTGCCAAAG CTTTTAAATT TGACGTGTAC AAAGTAACGC GTTACTTCGC
   401 AGTGCTTACA AATGCGGCTA ATCTTAGGTA TGCCAGCTTC GTATTTCCAA
   451 AAGTACAGCT CAGGAT
(15) INFORMATION FOR SEQ ID N°: 15
   (i) SEQUENCE CHARACTERISTICS :
      (a) LENGTH : 377 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°15 :
   1 CTCGTCCACA CATTCTCCTA AAATGCAAGC CTTTTTTTTC CCACAAGGTG
   51  TACCGTCGAC TACACTGAGT CTCCAATAAA TATGTTTTCC GGTGCAATTT
   101  ACCTTGCAGT CTTTGACGCC GTATGTAGGG TCAGCGTGCA TGCCTTCGTC
   151  GTACATATAC ACCCTCTGAC AGTAGTTGCT CAGTGTTGTC ATCCTACCAG
   201  GAAGCTTAGA CGAACGTTTT ATTGTTTTTG TCGTGTATCG TTCTCTAAGG
   251  CATTTGAATT CCGGACGGTT GTAGAGGTTC CTGACTTCTC GCTGGCAGCA
   301  ATAAGAGAAC TGATACTGGC GCTCGTCTTG CATCTTGTAA CTCATGAGGT
   351  ATCCGTCATC CCATGGGCAG TCCGCAG
(16) INFORMATION FOR SEQ ID N°: 16
   (i) SEQUENCE CHARACTERISTICS :
      (a) LENGTH : 1670 base pairs
      (b) TYPE : nucleic acid
      (c) STRANDEDNESS : single
      (d) TOPOLOGY : linear
   (ii) MOLECULE TYPE : cDNA
   (iii) FEATURE :
      (a) NAME/KEY : CDS
      (b) LOCATION : 54 .. 1520
   (iv) SEQUENCE DESCRIPTION : SEQ ID N° 16 :
   1 AAGGAAGAAG TTAGGCGTAG GCTTTGGGAA ACCGGTCATC CTCGAAACCA GAG
   54 ATG TCG GGA CTC AGC CTG AAA TTG TGG ATT GTA GCG TTC TTT TCT
      Met Ser Gly Leu Ser Leu Lys Leu Trp Ile Val Ala Phe Phe Ser
   99 TTC TGC TTG GCC GAG AAA GAG CAT GGG ATC GTG TAC CCC AGG ATG CTT
      Phe Cys Leu Ala Glu Lys Glu His Gly Ile Val Tyr Pro Arg Met Leu
   147 GAA AGC AGA GCA GCA ACT GGA GAG AGA ATG CTT AAA ATC AAC GAT GAC
       Glu Ser Arg Ala Ala Thr Gly Glu Arg Met Leu Lys Ile Asn Asp Asp
   195 CTG ACG TTG ACG CTG CAG AAG AGT AAG GTC TTC GCT GAC GAC TTT CTC
       Leu Thr Leu Thr Leu Gln Lys Ser Lys Val Phe Ala Asp Asp Phe Leu
   243 TTC AGC ACG ACC GAC GGA ATT GAA CCT ATT GAT TAC TAC ATC AAA GCC
       Phe Ser Thr Thr Asp Gly Ile Glu Pro Ile Asp Tyr Tyr Ile Lys Ala
   291 GAA GAC GCT GAA CGT GAC ATC TAC CAC GAC GCA ACT CAC ATG GCA TCA
       Glu Asp Ala Glu Arg Asp Ile Tyr His Asp Ala Thr His Met Ala Ser
   339 GTA AGG GTA ACG GAC GAT GAT GGC GTG GAA GTG GAA GGA ATT CTT GGA
       Val Arg Val Thr Asp Asp Asp Gly Val Glu Val Glu Gly Ile Leu Gly
   387 GAG AGG CTT CGT GTT AAA CCT TTG CCG GCA ATG GCC CGC AGC AGC GAT
       Glu Arg Leu Arg Val Lys Pro Leu Pro Ala Met Ala Arg Ser Ser Asp
   435 GGC CTC AGA CCG CAT ATG TTG TAC GAA GTC GAC GCA CAC GAA AAC GGC
       Gly Leu Arg Pro His Met Leu Tyr Glu Val Asp Ala His Glu Asn Gly
   483 CGG CCA CAT GAT TAT GGT TCA CCG AAC ACA ACA AAT ACC CCC GTA GAG
       Arg Pro His Asp Tyr Gly Ser Pro Asn Thr Thr Asn Thr Pro Val Glu
   531 AGA AGA GCT GGA GGC ACA GAA CCC CAG ATG TAC AAG ATA CCA GCG GAA
       Arg Arg Ala Gly Gly Thr Glu Pro Gln Met Tyr Lys Ile Pro Ala Glu
   579 ATC TAT CCC GAA GTT TAC CTT GTG GCG GAT AGT GCC TTT GCC AAA GAA
       Ile Tyr Pro Glu Val Tyr Leu Val Ala Asp Ser Ala Phe Ala Lys Glu
   627 TTT AAC TTT GAT GTG AAC GCC GTT ACG CGT TAC TTC GCA GTG CTT ACA
       Phe Asn Phe Asp Val Asn Ala Val Thr Arg Tyr Phe Ala Val Leu Thr
   675 AAT GCG GCT AAT CTT AGG TAT GAA AGC TTC AAA TCT CCA AAG GTA CAG
       Asn Ala Ala Asn Leu Arg Tyr Glu Ser Phe Lys Ser Pro Lys Val Gln
   723 CTC AGG ATC GTT GGC ATA ACG ATG AAC AAA AAC CCA GCA GAC GAG CCA
       Leu Arg Ile Val Gly Ile Thr Met Asn Lys Asn Pro Ala Asp Glu Pro
   771 TAC ATT CAC AAT ATA CGG GGA TAT GAG CAG TAC CGG AAT ATT TTG TTT
       Tyr Ile His Asn Ile Arg Gly Tyr Glu Gln Tyr Arg Asn Ile Leu Phe
   819 AAG GAA ACA CTG GAG GAT TTC AAC ACT CAG ATG AAG TCA AAA CAT TTT
       Lys Glu Thr Leu Glu Asp Phe Asn Thr Gln Met Lys Ser Lys His Phe
   867 TAT CGT ACT GCC GAT ATC GTG TTT CTC GTG ACA GCA AAA AAT ATG TCC
       Tyr Arg Thr Ala Asp Ile Val Phe Leu Val Thr Ala Lys Asn Met Ser
   915 GAA TGG GTT GGT AGC ACA CTA CAA TCA TGG ACT GGC GGG TAC GCT TAC
       Glu Trp Val Gly Ser Thr Leu Gln Ser Trp Thr Gly Gly Tyr Ala Tyr
   963 GTA GGA ACA GCG TGT TCC GAA TGG AAA GTA GGA ATG TGT GAA GAC CGA
       Val Gly Thr Ala Cys Ser Glu Trp Lys Val Gly Met Cys Glu Asp Arg
   1011 CCG ACA AGC TAT TAC GGA GCT TAC GTT TTC GCC CAT GAG CTG GCG CAT
        Pro Thr Ser Tyr Tyr Gly Ala Tyr Val Phe Ala His Glu Leu Ala His
   1059 AAT TTG GGT TGT CAA CAC GAT GGA GAT GGT GCC AAT AGC TGG GTG AAA
        Asn Leu Gly Cys Gln His Asp Gly Asp Gly Ala Asn Ser Trp Val Lys
   1107 GGG CAC ATC GGA TCT GCG GAC TGC CCA TGG GAT GAC GGA TAC CTT ATG
        Gly His Ile Gly Ser Ala Asp Cys Pro Trp Asp Asp Gly Tyr Leu Met
   1155 AGC TAC AAG ATG GAA GAC GAG CGC CAG TAT AAG TTT TCT CCC TAC TGC
        Ser Tyr Lys Met Glu Asp Glu Arg Gln Tyr Lys Phe Ser Pro Tyr Cys
   1203 CAG AGA GAA GTC AGG AAC CTC TAC AGG CGT CCG GAA TTC AAA TGC CTC
        Gln Arg Glu Val Arg Asn Leu Tyr Arg Arg Pro Glu Phe Lys Cys Leu
   1251 ACT GAA CGA AAA GCG AAA AAA ACA ATC CGC TCG TCT AAG CTA CCT GGT
        Thr Glu Arg Lys Ala Lys Lys Thr Ile Arg Ser Ser Lys Leu Pro Gly
   1299 GTG ATG ACA TCA TCG AGC AAC TAT TGC CGG AGG GTG TAC ATG TAC GAA
        Val Met Thr Ser Ser Ser Asn Tyr Cys Arg Arg Val Tyr Met Tyr Glu
   1347 AAA GGC ATG CAC GCC GAC GAG GCA TaT GGC GTC AAG GAC TGC AGG GTA
        Lys Gly Met His Ala Asp Glu Ala Tyr Gly Val Lys Asp Cys Arg Val
   1395 AAA TGC ACC ACC ACA TCA AGA ATG TAT TGG CTA CTC GGT GTA GTC GAC
        Lys Cys Thr Thr Thr Ser Arg Met Tyr Trp Leu Leu Gly Val Val Asp
   1443 GGT ACA CCT TGC GGA AAT GGA AAG GCT TGC ATT CTT GGG AAA TGC AGG
        Gly Thr Pro Cys Gly Asn Gly Lys Ala Cys Ile Leu Gly Lys Cys Arg
   1491 AAC AAA ATC AAA ATA AGC AAG AAG GAC TGA GAGGTTGATA ATATCAAATT
        Asn Lys Ile Lys Ile Ser Lys Lys Asp End
   1541 AATCATGATA TTTCAACCAC ATGACTTCGT GCTCAACTGG TAGCCCCAAA TAAATTTTAA
   1601 AAAAAATCCC AATATGCGTG GTAGAAAAAG CAGCAAACAA TAAAACTTCT AAAAATGTCT
   1661 TGCAAAAATG
(17) INFORMATION FOR SEQ ID N°: 17
   (i) SEQUENCE CHARACTERISTICS :
      (a) LENGTH : 158 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°17 :
   1  CACCAGTGAT GCTTATTGTT GCACTGCACT TGTTGATAAT ATCCGGTCGT
   51  CGAATTGCAC TTCGGAACTT CCACTCCAAC TTGGCGAGCC GTGGATTTTG
   101  ACTTCTCGTG ATGCTCCACC AGACAGTTGC AGGACTTCAG CTGCCTAGAT
   151  GGAGCCTT
(18) INFORMATION FOR SEQ ID N°: 18
   (i) SEQUENCE CHARACTERISTICS :
      (a) LENGTH : 146 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°18 :
   1  CTGTTGTTGA ACTGAAATAA ATAACAAAAA AATCATAAAG NTGGAGGAAA
   51  GATGATCGAN TCCCCGCCCC TTGACAATCG TCCGATAAAA ACCAACTATA
   101  TTCNGTCCTT TTTACAAACA ATTCCAANTG TCTGACCGAA CCGCGA
(19) INFORMATION FOR SEQ ID N°: 19
   (i) SEQUENCE CHARACTERISTICS :
      (a) LENGTH : 140 base pairs
      (b) TYPE : nucleic acid
      (c) STRANDEDNESS: single
      (d) TOPOLOGY : linear
   (ii) MOLECULE TYPE : cDNA
   (iii) SEQUENCE DESCRIPTION : SEQ ID N° 19 :
   1 CTNGGACGAN GTCCTATGAC TTGCGCTTAN GTTTCTTAGT CTTCTTCGGT
   51 TTCTTCTTTT TTTGCTTCGG TTTTTCGGTG GGCGCAGGTG TATAGTCATC
   101 AGTGTCGGTG GGCCCATCCG AATGAGTTGT CAAATGACAT
(20) INFORMATION FOR SEQ ID N°: 20
   (i) SEQUENCE CHARACTERISTICS :
      (a) LENGTH : 143 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°20 :
   1 TGCCGAAAAA TAACGATGAT TTGACGTTGA CTCTGCAGAA GAGTAAGGTT
   51 TTCACCGACA GTTTTCTGTT TAGCACGACG AAGGATAACG AGCCTATCGA
   101 TTACTACGTG AGAGCCGAAG ATGCCGAACG AGACATATAT CAC
(21) INFORMATION FOR SEQ ID N°: 21
   (i) SEQUENCE CHARACTERISTICS :
      (a) LENGTH : 140 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°21 :
   1 TGTTGCTACA GACTCGACGT TTCGAGCTTG CTCGCCATTT MAAGACAACG
   51 CACTCACAGA ATATTTAAGT GCGTTCGTGA WAGCTGTGGG CTTACGATTG
   101 CAGGCGCTTC ANTCACCAGC TGTGATATTA MAGTTCCTAG
(22) INFORMATION FOR SEQ ID N°: 22
   (i) SEQUENCE CHARACTERISTICS :
      (a) LENGTH : 144 base pairs
      (b) TYPE: nucleic acid
      (c) STRANDEDNESS : single
      (d) TOPOLOGY: linear
   (ii) MOLECULE TYPE : cDNA
   (iii) SEQUENCE DESCRIPTION : SEQ ID N°22 :
   1  TCACGATAGT TGAAACGTTG AAACTTGAAA TACTCCCACA GTCGTTGGAT
   51  GCTTCAGAAC TGCTAAGAAC TTCACACTTT GCAAGAAGTW CCAAAATGAA
   101  AGCCGCGATG ACCGATGATT TAGCTTCCAT CTTCTATCAC TTGA
(23) INFORMATION FOR SEQ ID N°: 23
   (i) SEQUENCE CHARACTERISTICS :
      (a) LENGTH : 95 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°23 :
   1  GACCACCCCG TCCGAACTTG CTAAAKCAAG CAATGGAGTG AGGTGTTCTA
   51  TGCGGGTTGA TTACACCAAT GGCGCTGCGT GGTGCGTGGT GATTT
(24) INFORMATION FOR SEQ ID N°: 24
   (i) SEQUENCE CHARACTERISTICS :
      (a) LENGTH : 1414 base pairs
      (b) TYPE : nucleic acid
      (c) STRANDEDNESS : single
      (d) TOPOLOGY : linear
   (ii) MOLECULE TYPE : cDNA
   (iii) FEATURE :
      (a) NAME/KEY : CDS
      (b) LOCATION : 143 1276
   (iv) SEQUENCE DESCRIPTION : SEQ ID N°24 :
   1 GTAGGGCCGT GCAAGCGAAG GCAGCGAAGG CTGCGAGTGT ACGTGCAGTT CGGAAGTGCA
   61 ATATCCTGTT ATTAAGCTCT AATTAGCACA CTGTGAGTCG ATCAGAGGCC TCTCTTAACG
   121 CCACATTGAA AAAGGATCCA AG ATG GAG GCA AGT CTG AGC AaC CAC ATC CTT
                                Met Glu Ala Ser Leu Ser Asn His Ile Leu
   173 AAC TTC TCC GTC GAC CTA TAC AAG CAG CTG AAA CCC TCC GGC AAA GAC
       Asn Phe Ser Val Asp Leu Tyr Lys Gln Leu Lys Pro Ser Gly Lys Asp
   221 ACG GCA GGA AAC GTC TTC TGC TCA CCA TTC AGT ATT GCA GCT GCT CTG
       Thr Ala Gly Asn Val Phe Cys Ser Pro Phe Ser Ile Ala Ala Ala Leu
   269 TCC ATG GCC CTC GCA GGA GCT AGA GGC AAC ACT GCC AAG CAA ATC GCT
       Ser Met Ala Leu Ala Gly Ala Arg Gly Asn Thr Ala Lys Gln Ile Ala
   317 GCC ATC CTG CAC TCA AAC GAC GAC AAG ATC CAC GAC CAC TTC TCC AAC
       Ala Ile Leu His Ser Asn Asp Asp Lys Ile His Asp His Phe Ser Asn
   365 TTC CTT TGC AAG CTT CCC AGT TAC GCC CCA GAT GTG GCC CTG CAC ATC
       Phe Leu Cys Lys Leu Pro Ser Tyr Ala Pro Asp Val Ala Leu His Ile
   413 GCC AAT CGC ATG TAC TCT GAG CAG ACC TTC CAT CCG AAA GCG GAG TAC
       Ala Asn Arg Met Tyr Ser Glu Gln Thr Phe His Pro Lys Ala Glu Tyr
   461 ACA ACC CTG TTG CAA AAG TCC TAC GAC AGC ACC ATC AAG GCT GTT GAC
       Thr Thr Leu Leu Gln Lys Ser Tyr Asp Ser Thr Ile Lys Ala Val Asp
   509 TTT GCA GGA AAT GCC GAC AGG GTC CGT CTG GAG GTC AAT GCC TGG GTT
       Phe Ala Gly Asn Ala Asp Arg Val Arg Leu Glu Val Asn Ala Trp Val
   557 GAG GAA GTC ACC AGG TCA AAG ATC AGG GAC CTG CTC GCA CCT GGA ACT
       Glu Glu Val Thr Arg Ser Lys Ile Arg Asp Leu Leu Ala Pro Gly Thr
   605 GTT GAT TCA TCG ACA TCA CTT ATA TTA GTG AAT GCC ATT TAC TTC AAA
       Val Asp Ser Ser Thr Ser Leu Ile Leu Val Asn Ala Ile Tyr Phe Lys
   653 GGT CTG TGG GAT TCT CAG TTC AAG CCT AGT GCT ACG AAG CCG GGA GAT
       Gly Leu Trp Asp Ser Gln Phe Lys Pro Ser Ala Thr Lys Pro Gly Asp
   701 TTT CAC TTG ACA CCA CAG ACC TCA AAG AAA GTG GAC ATG ATG CAC CAG
       Phe His Leu Thr Pro Gln Thr Ser Lys Lys Val Asp Met Met His Gln
   749 GAA GGG GAC TTC AAG ATG GGT CAC TGC AGC GAC CTC AAG GTC ACT GCG
       Glu Gly Asp Phe Lys Met Gly His Cys Ser Asp Leu Lys Val Thr Ala
   797 CTT GAG ATA CCC TAC AAA GGC AAC AAG ACG TCG ATG GTC ATT CTC CTG
       Leu Glu Ile Pro Tyr Lys Gly Asn Lys Thr Ser Met Val Ile Leu Leu
   845 CCC GAA GAT GTA GAG GGA CTC TCA GTC CTG GAG GAA CAC TTG ACC GCT
       Pro Glu Asp Val Glu Gly Leu Ser Val Leu Glu Glu His Leu Thr Ala
   893 CCG AAA CTG TCG GCT CTG CTC GGC GGC ATG TAT GCG ACG TCC GAT GTC
       Pro Lys Leu Ser Ala Leu Leu Gly Gly Met Tyr Ala Thr Ser Asp Val
   941 AAC TTG CGC TTG CCG AAG TTC AAA CTA GAG CAG TCC ATA GGT TTG AAG
       Asn Leu Arg Leu Pro Lys Phe Lys Leu Glu Gln Ser Ile Gly Leu Lys
   989 GAT GTA CTG ATG GCG ATG GGA GTC AAG GAT TTC TTC ACG TCC CTT GCA
       Asp Val Leu Met Ala Met Gly Val Lys Asp Phe Phe Thr Ser Leu Ala
   1037 GAT CTT TCT GGC ATC AGC GCT GCG GGG AAT CTG TGC GCT TCG GAT GTC
        Asp Leu Ser Gly Ile Ser Ala Ala Gly Asn Leu Cys Ala Ser Asp Val
   1085 ATC CAC AAG GCT TTT GTG GAA GTT AAT GAG GAG GGC ACA GAG GCT GCA
        Ile His Lys Ala Phe Val Glu Val Asn Glu Glu Gly Thr Glu Ala Ala
   1133 GCT GCC ACT GCC ATA CCC ATT ATG TTG ATG TGT GCG AGA TTT CCA CAG
        Ala Ala Thr Ala Ile Pro Ile Met Leu Met Cys Ala Arg Phe Pro Gln
   1181 GTG GTG AAC TTT TTC GTT GAC CGC CCA TTC ATG TTC TTG ATC CAC AGC
        Val Val Asn Phe Phe Val Asp Arg Pro Phe Met Phe Leu Ile His Ser
   1229 CAT GAT CCA GAT GTT GTT CTC TTC ATG GGA TCC ATC CGT GAG CTC TAA
   His Asp Pro Asp Val Val Leu Phe Met Gly Ser Ile Arg Glu Leu End
   1277 AAAGCATATT CTTAACGGCG GCCAATCAGT CTGTGGAGTT ATCTCTTAGT CACTAATGTG
   1337 TAACAATTCT GCAATATTCA GCTTGTGTAT TTCAGTAACT TGCTAGATCT TTGTGTTGTT
   1397 GATGTTAGGC TTCTTGCG
(25) INFORMATION FOR SEQ ID N°: 25
   (i) SEQUENCE CHARACTERISTICS :
      (a) LENGTH : 200 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°25 :
   1 ACCGTAACCA AAATTGTTTC TTTCCAGAAG AATGGTTCAA ACTTTTCAAA
   51 CAGATTTCGG AAACTCTTCT TGCACTTTTA AAATCCAATC TACAATCTTT
   101 CCTCGCACTT CTGAATTGCA TTCCAGTTTA CCTTCCAAGC AAACCTCTTT
   151 TGGCAACTCC AGCCGTACTC CATTTCGGCA TACCACAGTG CATGCACTTG
(26) INFORMATION FOR SEQ ID N°: 26
   (i) SEQUENCE CHARACTERISTICS :
      (a) LENGTH : 241 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION: SEQ ID N°26 :
   1 CGTATTCTTT GAAGATTTGT ATACGAAACA TAAATTCGTC ATGCATACTT
   51 TTGATGGTTA CACGACATGC GAAGCTGCCG ACAAAGAAGA CTGGGAAGAT
   101 AAGAAGCACC TAGTTACGGT AGTGCGTGGA CCGGATAAAC GAAAGTACAC
   151 GTTTCTACGC AACATTCTCA CCTTACAACG GAGAGTGAGA GTTAGCAAAA
   201 CAATGATTGA GCTCGTACGG AACATGTCCT GTAGGACATT T
(27) INFORMATION FOR SEQ ID N°: 27
   (i) SEQUENCE CHARACTERISTICS :
      (a) LENGTH : 313 base pairs
      (b) TYPE : nucleic acid
      (c) STRANDEDNESS : single
      (d) TOPOLOGY : linear
   (ii) MOLECULE TYPE : cDNA
   (iii) SEQUENCE DESCRIPTION : SEQ ID N°27 :
   1 AAGCANCCGG ACTACCTGCT TGAAAACGTT GTACGGGCAA ACTTGGACGG
   51 AAAACTCCCA GATGCTACTC CAGTTCCTCC CGGAAGCTAC ACGTACGCTG
   101 AGAATGATAA CTTCACCTGC TATTCCAGAA GTACACCGTT TCCGGATGGG
   151 GTGAATGTTG TATAACGGCT GCTGGGTGCG GAAGACTATG ATGGATTACG
   201 CAAAAAAGTT CTAAACGAGT TGTTTCCCAT CCCGGAAAGT CTGCTGTATG
   251 CTGACATGAT GCGACTTGTG GCTAAGAAAG ACAGAGTTGA TCACACTAGT
   301 GGATGACCTG GGA
(28) INFORMATION FOR SEQ ID N°: 28
   (i) SEQUENCE CHARACTERISTICS
      (a) LENGTH : 2417 base pairs
      (b) TYPE : nucleic acid
      (c) STRANDEDNESS : single
      (d) TOPOLOGY : linear
   (ii) MOLECULE TYPE : cDNA
   (iii) FEATURE:
      (a) NAME/KEY : CDS
      (b) LOCATION : 218 .. 1495
   (iv) SEQUENCE DESCRIPTION : SEQ ID N°28 :
   1 GTCGTAGTCG TAGTCGTAGT CAGTTGCGCA TGCGCGGGGC TTTCCTGTCT TTCTTGCCTT
   61 TCTGCAGTCG TTCACCAACA TGTGGATACA GCTCCGGAGA TTTGTAAACA AATACTGCAC
   121 TTTTAAGCAA GACTTGATAT TTAGATCGAT ATCCTCCTGT TGTCCGTCTT GATTAATCGG
   181 CTCTTTAGGG TTTTTAGAAT AGGCTTTTCG GTACGAG ATG CCC AAA GGA AAG AGG
                                           Met Pro Lys Gly Lys Arg
   236 GGA CCC AAA GCA GGT GGC GCC GCG CGC GGT GGC CGG TGC GAG GCC AGC
       Gly Pro Lys Ala Gly Gly Ala Ala Arg Gly Gly Arg Cys Glu Ala Ser
   284 CTG GCT CCG TCg TCC AGC GAC GAG GAG TCC AAC GCA GAC ACG GCG AGC
       Leu Ala Pro Ser Ser Ser Asp Glu Glu Ser Asn Ala Asp Thr Ala Ser
   332 GTG CTG AGC TGC GCC TCG GAG TCT CGC TGT GGC AGT GAC GGC ACC GTT
       Val Leu Ser Cys Ala Ser Glu Ser Arg Cys Gly Ser Asp Gly Thr Val
   380 GGA GAC CCA GAA GCG GAG GAG GCT GTG CTG CAT GAC GAC TTT GAA GAC
       Gly Asp Pro Glu Ala Glu Glu Ala Val Leu His Asp Asp Phe Glu Asp
   428 AAA CTC AAG GAG GCC ATC GAC GGA GCT TCG CAG AAG AGT GCC AAA GGA
       Lys Leu Lys Glu Ala Ile Asp Gly Ala Ser Gln Lys Ser Ala Lys Gly
   476 CGG CTG TCG TGC CTG GAG GCG ATT CGC AAG GCC TTT TcC ACC AAA TAC
       Arg Leu Ser Cys Leu Glu Ala Ile Arg Lys Ala Phe Ser Thr Lys Tyr
   524 CTG TAC GAC TTC CTC ATG GAC AGA CCG AGC ACG GTG TGC GAC CTG GTG
       Leu Tyr Asp Phe Leu Met Asp Arg Pro Ser Thr Val Cys Asp Leu Val
   572 GAG CGT GGG GTG CGC AAG GGC CGA GGG GAG GAG GCG GCC CTG TGC GCC
       Glu Arg Gly Val Arg Lys Gly Arg Gly Glu Glu Ala Ala Leu Cys Ala
   620 ACT CTC GGG GCC CTG GCC TGC GTC CAG CTC GGG GTC GGG GCC GAG GCG
       Thr Leu Gly Ala Leu Ala Cys Val Gln Leu Gly Val Gly Ala Glu Ala
   668 GAC GCC CTG TTC GAC GCC CTG CGC CAG CCG CTC TGC ACT TTG CTG CTT
       Asp Ala Leu Phe Asp Ala Leu Arg Gln Pro Leu Cys Thr Leu Leu Leu
   716 GAC GGG GCC CAG GGG CCC TCC CCC AGG GCC AGG TGT GCC ACT GCC CTC
        Asp Gly Ala Gln Gly Pro Ser Pro Arg Ala Arg Cys Ala Thr Ala Leu
   764 GGC CTC TGC TGc TTC GTG GTG GAC TCG GAC AAC CAG CTG GTG CTG CAG
       Gly Leu Cys Cys Phe Val Val Asp Ser Asp Asn Gln Leu Val Leu Gln
   812 CCG TGC ATG GAG GTG CTC TGG CAG GTG GTG GGT GCC AAG GCG GGC CCC
       Pro Cys Met Glu Val Leu Trp Gln Val Val Gly Ala Lys Ala Gly Pro
   860 GGC TCT CCG GTG CTC CAG GCA GCG GCC CTG CTC GCC TGG GGC CTC CTG
       Gly Ser Pro Val Leu Gln Ala Ala Ala Leu Leu Ala Trp Gly Leu Leu
   908 CTC AGC GTG GCT CCC GTC GAC CGc CTG CTG GCG CTc ACG CGC ACG CAC
       Leu Ser Val Ala Pro Val Asp Arg Leu Leu Ala Leu Thr Arg Thr His
   956 CTG CCC CGG CTG CAG GAG CTG CTG GAG AGC CCC GAC CTG GAC CTG CGC
       Leu Pro Arg Leu Gln Glu Leu Leu Glu Ser Pro Asp Leu Asp Leu Arg
   1004 ATT GCG GCC GGG GAG GTG ATC GCC GTC ATG TAC GAG GGG GCC AGG GAC
        Ile Ala Ala Gly Glu Val Ile Ala Val Met Tyr Glu Gly Ala Arg Asp
   1052 TAC GAC GAG GAC TTT GAG GAG CCC TCG GAG TCC CTG TGT GCC CAG CTG
        Tyr Asp Glu Asp Phe Glu Glu Pro Ser Glu Ser Leu Cys Ala Gln Leu
   1100 CGC CAG CTG GCC ACG GAC AGC CAG AAG TTT CGG GCC AAG AAG GAG CGG
        Arg Gln Leu Ala Thr Asp Ser Gln Lys Phe Arg Ala Lys Lys Glu Arg
   1148 CGC CAG CAG CGC TCC ACC TTC AGG GAC GTC TAC CGG GCC GTC AGG GAG
        Arg Gln Gln Arg Ser Thr Phe Arg Asp Val Tyr Arg Ala Val Arg Glu
   1196 GGG GCC TCT CCC GAC GTG AGC GTC AAG TTT GGC CGG GAA GTC CTG GAA
        Gly Ala Ser Pro Asp Val Ser Val Lys Phe Gly Arg Glu Val Leu Glu
   1244 CTG GAC ACC TGG AGT CGC AAG CTG CAG TAC GAC GCT TTC TGC CAG CTG
        Leu Asp Thr Trp Ser Arg Lys Leu Gln Tyr Asp Ala Phe Cys Gln Leu
   1292 CTG GGC TCC GGC ATG AAC CTG CAC CTG GCC GTG AAC GAG CTG CTG AGG
        Leu Gly Ser Gly Met Asn Leu His Leu Ala Val Asn Glu Leu Leu Arg
   1340 GAC ATC TTT GAA CTG GGG CAG GTG CTG GCA ACC GAG GAC CAC ATT ATC
        Asp Ile Phe Glu Leu Gly Gln Val Leu Ala Thr Glu Asp His Ile Ile
   1388 TCC AAG ATC ACC AAG TTC GAA AGG CAC ATG GTG AAC ATG GCC AGC TGC
        Ser Lys Ile Thr Lys Phe Glu Arg His Met Val Asn Met Ala Ser Cys
   1436 CGG GCC CGC ACC AAG ACA CGC AAC CGG CTG AGG GAC AAG CGC GCC GAC
        Arg Ala Arg Thr Lys Thr Arg Asn Arg Leu Arg Asp Lys Arg Ala Asp
   1484 GTG GTC GCC TGA ACCTGCGGAG GGATGCTTAG CTATGCACTC GCCGGCCTAC
        Val Val Ala End
   1536 CCTGGCGGGA CTCGATGCCA CTCACGAGTC GGCGCTCGCA AATTCGCCGC CCATCGTTAC
   1596 GCAATGGGAG ACAAAGCTGC TTTTGGCATT ACCGTTTGAG GTCGGCTCCA ACCCATAGAT
   1656 GAATTTCTTT TTTGTGGCCG TTTCTGGGTT ACATGTTTTG GGGGAAGGGA GTGGAACTGT
   1716 CCGGTTCTTT GGCACACGTC AGGTTGCTCT TGATGCGCGA CGTGCTTGTA TTTGGGtACT
   1776 GCCGACACCA AGCGTTTCGG CGATTCCTGG AAAAGAGTGC CTCTCGCTCG ACGTTTGGTT
   1836 GTTTTCTGCG TGGTCCGTCG TCGACCTTCG TTCGTCCAAA GACGCCGTCC GGTTTCAtAC
   1896 TCCCCCCCGC ACACATATCG AGGCCAATTA AATTGCTAAG GGTGCCGTTG TCGTGCATCT
   1956 GGCAGGCTCA GAAGTGGCTT ATTTGTCTTT TAATTTTGCC GATGCACGCA AAAATTGTCA
   2016 TTTCTTGAAA GTTTCTCTTT TATTGCGTAC ACAATTCAAC TTTTATGTAA TTTCTGATGG
   2076 TCTGTTTTAC GTGTGCGTGT GTAAAACGTA ACTTTGGAAG AATTTTTATG CACACTGAAC
   2136 AAACGCTCGG TCCTGGGGTT GAAAGTGCTC GGTGTGTGCA TGAGCTAAAG TGCAACTGCT
   2196 TTGTTCCGAA GGTTTTCTAG TCGCCGAAAT GTACCATTGT GGACCTTGTT GCGAGAGACC
   2256 TTGGTCTTCT GGGGGAGCTG CTGTAGCGTG GCAAGCCACT ATTTTGGGAG CGACATTGCA
   2316 GAGAAAATCG GCTTTTAGAA AGGCACCTGC GCGGCGAGTG GACGTTTTTT CGTATATACT
   2376 GCGAAAAAAA AAAAAAAAAA AAAAAAAAAA AAAAAAAAAA AA
(29) INFORMATION FOR SEQ ID N°: 29
   (i) SEQUENCE CHARACTERISTICS :
      (a) LENGTH : 933 base pairs
      (b) TYPE : nucleic acid
      (c) STRANDEDNESS : single
      (d) TOPOLOGY : linear
   (ii) MOLECULE TYPE : cDNA
   (iii) FEATURE :
      (a) NAME/KEY : CDS
      (b) LOCATION : 32 .. 853
   (iv) SEQUENCE DESCRIPTION : SEQ ID N°29 :

### Bibliography.

Ganapamo et al, 1997 : Identification of an Ixodes ricinus salivary gland fraction through its ability to stimulate CD4 T cells present in BALB/c mice lymph nodes draining the tick fixation site. Ganapamo-F; Rutti-B; Brossard-M. Parasitology; 1997 Jul; 115 (Pt 1): 91-6
Ganapamo et al, 1995 : In vitro production of interleukin-4 and interferon-gamma by lymph node cells from BALB/c mice infested with nymphal Ixodes ricinus ticks. Ganapamo-F; Rutti-B; Brossard-M. Immunology; 1995 May; 85(1): 120-4
Ganapamo et al, 1996 : Immunosuppression and cytokine production in mice infested with Ixodes ricinus ticks: a possible role of laminin and interleukin-10 on the in vitro responsiveness of lymphocytes to mitogens. Ganapamo-F; Rutti-B; Brossard-M. Immunology; 1996 Feb; 87(2): 259-63
Wikel et al. 1996 : Host immunity to ticks. Wikel-SK. Annu-Rev-Entomol.;. 1996; 41: 1-22
Wikel and Brossard, 1997 : Immunology of interactions between ticks and hosts. Brossard-M; Wikel-SK. Med-Vet-Entomol.; 1997 Jul; 11(3): 270-6
De Silva et al, 1995 : Growth and migration of Borrelia burgdorferi in Ixodes ticks during blooed feeding. Aravinda M De Silva, Erol Fikrig. Am. J. Trop. Med. Hyg.; 53(4), 1995 pp 397-404
Hubank and Schatz, 1994: Identifying differences in mRNA expression by representational difference analysis of cDNA. Hubank-M; Schatz-DG. Nucleic-Acids-Res.; 1994 Dec 25; 22(25): 5640-8
Frohman, 1995 : Rapid amplification of cDNA Ends. In PCR Primer. A laboratory manual (Dieffenbach, C. W. and Dveksler, G. S., eds), pp.381-409, Cold Spring Harbor Laboratory Press, Cold Spring Harbor, NY.

## Claims

1. A polynucleotide obtained from tick salivary gland and presenting more than 75% identity with the nucleotide sequence SEQ.ID.NO.24 or a sequence complementary thereto.

2. The polynucleotide according to claim 1, which presents at least 80% identity with SEQ.ID.NO.24 nucleotide sequence or a sequence complementary thereto.

3. The polynucleotide according to claim 1 or 2, which is at least 90% identical with SEQ.ID.NO.24 nucleotide sequence or a sequence complementary thereto.

4. The polynucleotide according to any one of the preceding claims 1 to 3, which is at least 95% identical with SEQ.ID.NO.24 nucleotide sequence or a sequence complementary thereto.

5. The polynucleotide according to any one of the preceding claims 1. to 4, which is at least 98-99% identical with SEQ.ID.NO-24 nucleotide sequence or a sequence complementary thereto.

6. The polynucleotide according to any one of the preceding claims 1 to 5, which is at least 99% identical with SEQ.ID.NO.24 nucleotide sequence or a sequence complementary thereto.

7. A polynucleotide having the nucleotide sequence SEQ.ID.NO.24 or a sequence complementary thereto

8. A tick Salivary gland polypeptide encoded by the polynucleotide according to any one of the preceding claims 1 to 7 ,

9. A polypeptide according to claim 8, modified by or linked to at least one substitution group, preferably selected from the group consisting of amide, acetyl, phosphoryl, and/or glycosyl groups.

10. The polypeptide according to claim 8 or 9 in the form of a "mature" protein.

11. The polypeptide according to claim 8 or 9 as part of a larger protein.

12. The polypeptide according to claim 8 or 9 as part of a fusion protein.

13. The polypeptide according to any one of the preceding claims 8 to 12 further including at least one additional amino acid sequence which contains secretor or leader sequences, pro-sequences, sequences which help in purification such as multiple histidine residues, or additional sequences for stability during recombination protection.

14. A vector comprising at least one element selected from the group consisting of the polynucleotide according to any one of the preceding claims 1 to 7 or the polypeptide according to any one of the preceding claims 8 to 13.

15. A cell line transfected by the vector according to claim 14.

16. An antibody raised against *Ixodes ricinus* polypeptide according to any of the preceding claims 8 to 13.

17. A hybridoma cell line expressing the antibody according to the claim 16.

18. A pharmaceutical composition comprising an adequate pharmaceutical carrier and an element selected from the group consisting of the polynucleotide according to any of the preceding claims 1 to 7 or the polypeptide according to any of the preceding claims 8 to 13.

19. The pharmaceutical composition according to claim 18 which presents immunomodulatory properties.

20. An immunological composition or vaccine for inducing an immunological response in a mammalian host to a tick salivary gland polypeptide which comprises at least one element of the group consisting of :
a) a tick salivary gland cDNA according to any of the preceding claims 1 to 7,
b) a tick salivary gland polypeptide according to any of the preceding claims 8 to 13,
c) epitope-bearing fragments, outer-membrane vesicles or cells (attenuated or otherwise) of components a) or b); and
d) Possibly a carrier.

21. Use of the immunological composition a vaccine according to claim 20 or the vector according to the claim 14 for the manufacture of a medicament to produce antibody and/or T-cell immune response to protect a mammal from bacteria and viruses transmitted during the blood meal of *Ixodes ricinus* and related species and in the treatment or the prevention of lyme diseases or tick encephalitis virus diseases.

22. A diagnostic kit for detecting a disease or susceptibility to a disease induced or transmitted by tick, especially *Ixodes ricinus*, which comprises:
a) an *Ixodes ricinus* salivary gland polynucleotide according to any of the preceding claims 1 to 7,
b) a nucleotide sequence complementary to that of a),
c) an *Ixodes ricinus* salivary gland polypeptide according to any one of the preceding claims 8 to 13,
d) the antibody according to claim 16,
e) A phage displaying the antibody according to claim 16

23. A polynucleotide which is identical to the nucleotide sequence according to any one of the preceding claims 1 to 7 for use as hybridisation probe for cDNA clones encoding ticks, more particularly *Ixodes ricinus* salivary gland polypeptide, or for isolating clones or other genes similar to tick salivary gland cDNAs.

## Patentansprüche

1. Polynukleotid, das aus der Speicheldrüse einer Zecke erhalten wird und zu mehr als 75% mit der Nukleotidsequenz SEQ. ID. NO. 24 oder einer dazu komplementären Sequenz identisch ist.

2. Polynukleotid nach Anspruch 1, welches zu mindestens 80% mit der Nukleotidsequenz SEQ.ID.NO. 24 oder einer dazu komplementären Sequenz identisch ist.

3. Polynukleotid nach Anspruch 1 oder 2, welches zu mindestens 90% mit der Nukleotidsequenz SEQ.ID.NO. 24 oder einer dazu komplementären Sequenz identisch ist.

4. Polynukleotid nach einem beliebigen der vorherigen Ansprüche 1 bis 3, welches zu mindestens 95% mit der Nukleotidsequenz SEQ.ID.NO. 24 oder einer dazu komplementären Sequenz identisch ist.

5. Polynukleotid nach einem beliebigen der vorherigen Ansprüche 1 bis 4, welches zu mindestens 98-99% mit der Nukleotidsequenz SEQ.ID.NO. 24 oder einer dazu komplementären Sequenz identisch ist.

6. Polynukleotid nach einem beliebigen der vorherigen Ansprüche 1 bis 5, welches zu mindestens 99% mit der Nukleotidsequenz SEQ.ID.NO. 24 oder einer dazu komplementären Sequenz identisch ist.

7. Polynukleotid mit der Nukleotidsequenz SEQ.ID.NO. 24 oder einer dazu komplementären Sequenz.

8. Polypeptid aus der Speicheldrüse einer Zecke, das von dem Polynukleotid nach einem der vorherigen Ansprüche 1 bis 7 kodiert wird.

9. Polypeptid nach Anspruch 8, modifiziert von oder gekoppelt mit mindestens einer Substitutionsgruppe, die vorzugsweise aus der Gruppe ausgewählt ist, die aus Amid-, Acetyl-, Phosphoryl- und/oder Glycosylgruppen besteht.

10. Polypeptid nach Anspruch 8 oder 9 in Form eines "reifen" Proteins.

11. Polypeptid nach Anspruch 8 oder 9 als Teil eines größeren Proteins.

12. Polypeptid nach Anspruch 8 oder 9 als Teil eines Fusionsproteins.

13. Polypeptid nach einem beliebigen der vorherigen Ansprüche 8 bis 12, das des Weiteren mindestens eine zusätzliche Aminosäuresequenz aufweist, die Sekretor- oder Leitsequenzen, Prosequenzen, Sequenzen, die bei der Reinigung helfen, wie beispielsweise zahlreiche Histidinreste, oder zusätzliche Sequenzen für Stabilität während des Rekombinationsschutzes enthält.

14. Vektor, der mindestens ein Element aufweist, welches aus der Gruppe ausgewählt ist, die aus dem Polynukleotid nach einem beliebigen der vorherigen Ansprüche 1 bis 7 oder dem Polypeptid nach einem beliebigen der vorherigen Ansprüche 8 bis 13 besteht.

15. Zelllinie, die mit dem Vektor nach Anspruch 14 transfiziert ist.

16. Antikörper, der gegen Ixodes ricinus-Polypeptid nach einem beliebigen der vorherigen Ansprüche 8 bis 13 gerichtet ist.

17. Hybridoma-Zelllinie, die den Antikörper nach Anspruch 16 exprimiert.

18. Pharmazeutische Zusammensetzung, die einen geeigneten pharmazeutischen Träger und ein Element aufweist, welches aus der Gruppe ausgewählt ist, die aus dem Polynukleotid nach einem beliebigen der vorherigen Ansprüche 1 bis 7 oder dem Polypeptid nach einem beliebigen der vorherigen Ansprüche 8 bis 13 besteht.

19. Pharmazeutische Zusammensetzung nach Anspruch 18, welche immunmodulatorische Eigenschaften aufweist.

20. Immunologische Zusammensetzung oder Impfstoff zum Einführen einer immunologischen Reaktion gegen ein Polypeptid aus der Speicheldrüse einer Zecke in einen Säugerwirt, welche mindestens ein Element aus der Gruppe aufweist, die aus Folgendem besteht:
a) einer cDNA aus der Speicheldrüse einer Zecke nach einem beliebigen der vorherigen Ansprüche 1 bis 7,
b) einem Polypeptid aus der Speicheldrüse einer Zecke nach einem beliebigen der vorherigen Ansprüche 8 bis 13,
c) Epitop tragenden Fragmenten, Außenmembranvesikeln oder Zellen (abgeschwächt oder andersartig) von Komponente a) oder b); und
d) Möglichweise einem Träger.

21. Verwendung der immunologischen Zusammensetzung mit einem Impfstoff nach Anspruch 20 oder dem Vektor nach Anspruch 14 für die Herstellung eines Medikamentes zur Erzeugung von Antikörper- und/oder T-Zell-Immunreaktionen zum Schutz eines Säugers vor Bakterien und Viren, die während der Blutmahlzeit von *Ixodes ricinus* und verwandten Arten übertragen werden, und zur Behandlung oder zur Vorbeugung von Lyme-Krankheiten oder durch Zecken übertragener viraler Enzephalitiserkrankungen.

22. Diagnosekit zum Nachweis einer Krankheit oder Anfälligkeit für eine Krankheit, die durch Zecken, insbesondere durch *Ixodes ricinus,* ausgelöst oder übertragen wird, welches Folgendes umfasst:
a) ein Polynukleotid aus der Speicheldrüse von *Ixodes ricinus* nach einem beliebigen der vorherigen Ansprüche 1 bis 7;
b) eine zu der von a) komplementäre Nukleotidsequenz;
c) ein Polypeptid aus der Speicheldrüse von *Ixodes ricinus* nach einem beliebigen der vorherigen Ansprüche 8 bis 13;
d) den Antikörper nach Anspruch 16;
e) ein Phage, der den Antikörper nach Anspruch 16 präsentiert.

23. Polynukleotid, das zu der Nukleotidsequenz nach einem beliebigen der vorherigen Ansprüche 1 bis 7 identisch ist, zur Verwendung als Hybridisierungssonde für cDNA-Klone, die ein Polypeptid aus der Speicheldrüse einer Zecke, insbesondere *Ixodes ricinus,* kodieren, oder zum Isolieren von Klonen oder anderen Genen, die den cDNAs aus der Speicheldrüse einer Zecke ähnlich sind.

## Revendications

1. Polynucléotide obtenu à partir de glande salivaire de tique et présentant plus de 75 % d'identité avec la séquence nucléotidique SEQ ID N° 24 ou une séquence complémentaire de celle-ci.

2. Polynucléotide selon la revendication 1, qui présente au moins 80 % d'identité avec la séquence nucléotidique SEQ ID N° 24 ou une séquence complémentaire de celle-ci.

3. Polynucléotide selon la revendication 1 ou 2, qui est identique au moins à 90 % à la séquence nucléotidique SEQ ID N° 24 ou à une séquence complémentaire de celle-ci.

4. Polynucléotide selon l'une quelconque des revendications précédentes 1 à 3, qui est identique au moins à 95 % à la séquence nucléotidique SEQ ID N° 24 ou à une séquence complémentaire de celle-ci.

5. Polynucléotide selon l'une quelconque des revendications précédentes 1 à 4, qui est identique au moins à 98-99 % à la séquence nucléotidique SEQ ID N° 24 ou à une séquence complémentaire de celle-ci.

6. Polynucléotide selon l'une quelconque des revendications précédentes 1 à 5, qui est identique au moins à 99 % à la séquence nucléotidique SEQ ID N° 24 ou à une séquence complémentaire de celle-ci.

7. Polynucléotide ayant la séquence nucléotidique de SEQ ID N° 24 ou une séquence complémentaire de celle-ci.

8. Polypeptide de glande salivaire de tique codée par le polynucléotide selon l'une quelconque des revendications précédentes 1 à 7.

9. Polypeptide selon la revendication 8, modifié par ou lié à au moins un groupe de substitution, de préférence choisi dans le groupe composé des groupes amide, acétyle, phosphoryle, et/ou glycosyle.

10. Polypeptide selon la revendication 8 ou 9 sous la forme d'une protéine « mature ».

11. Polypeptide selon la revendication 8 ou 9 comme élément d'une protéine plus grande.

12. Polypeptide selon la revendication 8 ou 9 comme élément d'une protéine de fusion.

13. Polypeptide selon l'une quelconque des revendications précédentes 8 à 12 incluant en outre au moins une séquence d'acides aminés additionnelle qui contient des séquences sécrétrices ou de tête, des proséquences, des séquences qui facilitent la purification comme des résidus histidine multiples, ou des séquences additionnelles assurant la stabilité pendant la protection de recombinaison.

14. Vecteur comprenant au moins un élément choisi dans le groupe composé du polynucléotide selon l'une quelconque des revendications précédentes 1 à 7 ou du polypeptide selon l'une quelconque des revendications précédentes 8 à 13.

15. Lignée de cellules transfectée par le vecteur selon la revendication 14.

16. Anticorps dirigé contre le polypeptide d'*Ixodes ricinus* selon l'une quelconque des revendications précédentes 8 à 13.

17. Lignée de cellules hybridomes exprimant l'anticorps selon la revendication 16.

18. Composition pharmaceutique comprenant un véhicule pharmaceutique adéquat et un élément choisi dans le groupe composé du polynucléotide selon l'une quelconque des revendications précédentes 1 à 7 ou du polypeptide selon l'une quelconque des revendications précédentes 8 à 13.

19. Composition pharmaceutique selon la revendication 18 qui présente des propriétés immunomodulatrices.

20. Composition immunologique ou vaccin pour provoquer chez un hôte mammifère une réponse immunologique à un polypeptide de glande salivaire de tique qui comprend au moins un élément du groupe composé de :
a) un ADNc de glande salivaire de tique selon l'une quelconque des revendications précédentes 1 à 7,
b) un polypeptide de glande salivaire de tique selon l'une quelconque des revendications précédentes 8 à 13,
c) des fragments porteurs d'épitope, des vésicules de membrane externe ou des cellules (atténués ou autres) de composants a) ou b) ; et
d) éventuellement un véhicule.

21. Utilisation de la composition immunologique, d'un vaccin selon la revendication 20 ou du vecteur selon la revendication 14 pour fabriquer un médicament destiné à produire un anticorps et/ou une réponse immunitaire par lymphocyte T pour protéger un mammifère contre des bactéries et des virus transmis pendant la piqûre d'Ixodes *ricinus* et d'espèces apparentées et dans le traitement ou la prévention de la maladie de Lyme ou des maladies liées au virus de l'encéphalite à tique.

22. Kit de diagnostic pour détecter une maladie ou une prédisposition à une maladie induite ou transmise par les tiques, en particulier par *Ixodes ricinus,* qui comprend :
a) un polynucléotide de glande salivaire d'Ixodes *ricinus* selon l'une quelconque des revendications précédentes 1 à 7,
b) une séquence nucléotidique complémentaire de celle de a),
c) un polypeptide de glande salivaire d'*Ixodes ricinus* selon l'une quelconque des revendications précédentes 8 à 13,
d) l'anticorps selon la revendication 16,
e) un phage présentant l'anticorps selon la revendication 16.

23. Polynucléotide identique à la séquence nucléotidique selon l'une quelconque des revendications précédentes 1 à 7 à utiliser comme sonde d'hybridation pour des clones d'ADNc codant un polypeptide de glande salivaire de tique, plus particulièrement d'Ixodes *ricinus,* ou pour isoler des clones ou d'autres gènes similaires aux ADNc de glande salivaire de tique.
